# EUROPEAN PATENT APPLICATION

(11) **EP 4 748 827 A1**
(43) Date of publication of application: **27.05.2026**
(21) Application number: 24843164.5
(22) Date of filing: 18.07.2024
(51) Int. Cl.: C07C 69/734, A01N 37/36

(54) **DIMETHYLPENTA-2-ENEDIOATE COMPOUND AND USE THEREOF**

(30) Priority: 19.07.2023 JP 2023117357
(71) Applicant: Sumitomo Chemical Company, Limited, Tokyo 103-6020 (JP)
(72) Inventor: OKURA, Yuka, Takarazuka-shi, Hyogo 665-8555 (JP)
(74) Representative: Vossius & Partner Patentanwälte Rechtsanwälte mbB
(86) International application number: PCT/JP2024/025746
(87) International publication number: WO 2025/018375

(57) **Abstract**

The present invention provides a compound having excellent control efficacy against plant diseases. A compound represented by formula (A) 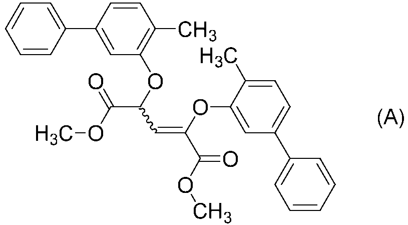 can be used for controlling plant diseases.

## Description

### TECHNICAL FIELD

This application claims the priority to and the benefit of Japanese Patent Application No. 2023-117357 filed on July 19, 2023, the entire contents of which are incorporated herein by reference.

The present invention relates to a dimethylpent-2-enedioate compound and use thereof.

### BACKGROUND ART

Non-Patent Document 1 discloses compounds having control effects against plant diseases.

### CITATION LIST

### NON-PATENT DOCUMENT

Non-Patent Document 1: The Pesticide Manual-18th Edition (published by BCPC); ISBN 978-1-9998966-1-4

### SUMMARY OF THE INVENTION

### PROBLEMS TO BE SOLVED BY THE INVENTION

An object of the present invention is to provide a compound having excellent control efficacy against plant diseases.

### MEANS TO SOLVE PROBLEMS

The present inventor has studied the above problems. As a result, she has found that a compound represented by the following formula (A) has excellent control efficacy against plant diseases, and completed the present invention.

Namely, the present invention provides the followings.
[1] A compound represented by formula (A) 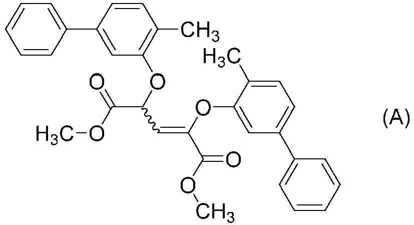 (hereinafter referred to as "Present compound" or "Compound of the present invention").
[2] A composition for controlling a plant disease comprising the compound according to [1] and an inert carrier (hereinafter referred to as "Present composition for controlling a plant disease" or "Composition for controlling a plant disease of the present invention").
[3] A composition comprising one or more ingredient(s) selected from the group consisting of Group (a), Group (b), Group (c), and Group (d), and the compound according to [1] :
   Group (a): a group consisting of insecticidal active ingredients, miticidal active ingredients, and nematicidal active ingredients;
   Group (b): fungicidal active ingredients;
   Group (c): plant growth regulatory ingredients;
   Group (d): repellent ingredients.
[4] A method for controlling a plant disease which comprises applying an effective amount of the compound according to [1], an effective amount of the composition for controlling a plant disease according to [2], or an effective amount of the composition according to [3], to a plant or soil for cultivating a plant (hereinafter referred to as "Present method for controlling a plant disease" or "Method for controlling a plant disease of the present invention").
[5] Use of the compound according to [1], the composition for controlling a plant disease according to [2], or the composition according to [3], for controlling a plant disease.
[6] A seed or a vegetative reproductive organ holding an effective amount of the compound according to [1], an effective amount of the composition for controlling a plant disease according to [2], or an effective amount of the composition according to [3].

### EFFECT OF INVENTION

The Present compound has excellent control efficacy against plant diseases, and is useful as an active ingredient of a composition for controlling plant diseases.

### MODE FOR CARRYING OUT THE INVENTION

The Present compound has two or more stereoisomers. Examples of the stereoisomers include diastereomers and enantiomers. The Present compound encompasses each stereoisomer and mixtures of stereoisomers at any ratio.

In the Present compound, the wavy line bound to the carbon-carbon double bond indicates that said wavy line may bind to said carbon-carbon double bond at either configuration, and the Present compound encompasses E isomer, Z isomer, and mixtures of E isomer and Z isomer at any ratio.

Examples of the stereoisomers include a compound represented by formula (1) 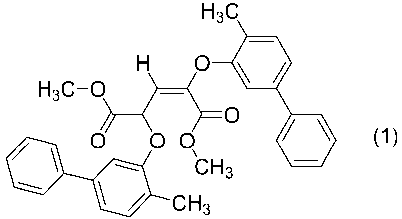 and a compound represented by formula (2) 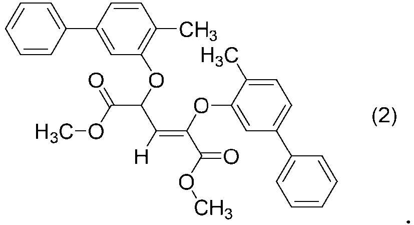 The compound represented by formula (1) and the compound represented by formula (2) are diastereomers with each other.

Further, each of the compound represented by formula (1) and the compound represented by formula (2) has an enantiomer.

Aspects of the Present compound include the following compounds.

The compound represented by formula (1).

The compound represented by formula (2).

A mixture of stereoisomers comprising a larger amount of the compound represented by formula (1) than the amount of the compound represented by formula (2).

A mixture of stereoisomers comprising a larger amount of the compound represented by formula (2) than the amount of the compound represented by formula (1).

A mixture of stereoisomers comprising the compound represented by formula (1) and the compound represented by formula (2) at a ratio of 1 : 1.

The Present compound may be mixed with or used in combination with one or more ingredient(s) selected from the group consisting of the following Group (a), Group (b), Group (c), and Group (d) (hereinafter referred to as "Present ingredient").

When the Present compound is mixed with or used in combination with the Present ingredient, they are used simultaneously, separately, or at time intervals with each other.

When the Present compound is used simultaneously with the Present ingredient, the Present compound and the Present ingredient may be contained in separate formulations or contained in one formulation.

One aspect of the present invention provides a composition comprising one or more ingredient (s) selected from the group consisting of Group (a), Group (b), Group (c), and Group (d) (i.e., Present ingredient), and the Present compound (hereinafter referred to as "Composition A").

Group (a) is a group consisting of acetylcholinesterase inhibitors (for example, carbamate insecticides and organophosphate insecticides), GABA-gated chloride channel blockers (for example, phenylpyrazole insecticides), sodium channel modulators (for example, pyrethroid insecticides), nicotinic acetylcholine receptor competitive modulators (for example, neonicotinoid insecticides), nicotinic acetylcholine receptor allosteric modulators, glutamate-gated chloride channel allosteric modulators (for example, macrolide insecticides), juvenile hormone mimics, multisite inhibitors, chordotonal organ TRPV channel modulators, mite growth inhibitors, microbial disruptors of insect midgut membranes, inhibitors of mitochondrial ATP synthase, uncouplers of oxidative phosphorylation, nicotinic acetylcholine receptor channel blockers (for example, nereistoxin insecticides), inhibitors of chitin biosynthesis, moulting disruptors, ecdysone receptor agonists, octopamine receptor agonists, mitochondrial complexes I, II, III, and IV electron transport inhibitors, voltage-dependent sodium channel blockers, inhibitors of acetyl CoA carboxylase, ryanodine receptor modulators (for example, diamide insecticides), chordotonal organ modulators, and microbial insecticides, and other insecticidal active ingredients, miticidal active ingredients, and nematicidal active ingredients. These ingredients are described in the classification on the basis of action mechanism by IRAC.

Group (b) is a group consisting of nucleic acids synthesis inhibitors (for example, phenylamide fungicides and acylamino acid fungicides), cell division and cytoskeleton inhibitors (for example, MBC fungicides), respiration inhibitors (for example, QoI fungicides and QiI fungicides), amino acids synthesis and protein synthesis inhibitors (for example, anilino-pyrimidine fungicides), signal transduction inhibitors, lipid synthesis and membrane synthesis inhibitors, sterol biosynthesis inhibitors (for example, DMI fungicides such as triazole fungicides), cell wall biosynthesis inhibitors, melanin synthesis inhibitors, plant defense inducers, fungicides with multi-site contact activity, microbial fungicides, and other fungicidal active ingredients. These ingredients are described in the classification on the basis of action mechanism by FRAC.

Group (c) is a group of plant growth regulatory ingredients (including mycorrhizal fungi and root nodule bacteria).

Group (d) is a group of repellent ingredients.

Hereinafter, examples of the combination of the Present ingredient and the Present compound are described. For example, "alanycarb + SX" indicates a combination of alanycarb and SX.

The abbreviation of "SX" indicates the Present compound. Also, all of the following Present ingredient are known ingredients, and may be obtained from commercially available formulations, or may be prepared by known methods. When the Present ingredient is a microorganism, it may also be available from a bacterial authority depository. Further, the number in parentheses represents the CAS RN (registered trademark).

Combinations of the Present ingredient in the above Group (a) and the Present compound:
abamectin + SX, acephate + SX, acequinocyl + SX, acetamiprid + SX, acetoprole + SX, acrinathrin + SX, acynonapyr + SX, afidopyropen + SX, afoxolaner + SX, alanycarb + SX, aldicarb + SX, allethrin + SX, alpha-cypermethrin + SX, alpha-endosulfan + SX, aluminium phosphide + SX, amitraz + SX, azadirachtin + SX, azamethiphos + SX, azinphos-ethyl + SX, azinphos-methyl + SX, azocyclotin + SX, bark of Celastrus angulatus + SX, bendiocarb + SX, benfluthrin + SX, benfuracarb + SX, bensultap + SX, benzoximate + SX, benzpyrimoxan + SX, beta-cyfluthrin + SX, beta-cypermethrin + SX, bifenazate + SX, bifenthrin + SX, bioallethrin + SX, bioresmethrin + SX, bistrifluron + SX, borax + SX, boric acid + SX, broflanilide + SX, bromopropylate + SX, buprofezin + SX, butocarboxim + SX, butoxycarboxim + SX, cadusafos + SX, calcium phosphide + SX, carbaryl + SX, carbofuran + SX, carbosulfan + SX, cartap hydrochloride + SX, cartap + SX, chinomethionat + SX, chlorantraniliprole + SX, chlordane + SX, chlorethoxyfos + SX, chlorfenapyr + SX, chlorfenvinphos + SX, chlorfluazuron + SX, chlormephos + SX, chloropicrin + SX, chlorpyrifos + SX, chlorpyrifos-methyl + SX, chromafenozide + SX, clofentezine + SX, clothianidin + SX, concanamycin A + SX, coumaphos + SX, cryolite + SX, cyanophos + SX, cyantraniliprole + SX, cyclaniliprole + SX, cyclobutrifluram + SX, cycloprothrin + SX, cycloxaprid + SX, cyenopyrafen + SX, cyetpyrafen + SX, cyflumetofen + SX, cyfluthrin + SX, cyhalodiamide + SX, cyhalothrin + SX, cyhexatin + SX, cypermethrin + SX, cyphenothrin + SX, cyproflanilide + SX, cyromazine + SX, dazomet + SX, deltamethrin + SX, demeton-S-methyl + SX, diafenthiuron + SX, diazinon + SX, dichlorvos + SX, dicloromezotiaz + SX, dicofol + SX, dicrotophos + SX, diflovidazin + SX, diflubenzuron + SX, dimefluthrin + SX, dimethoate + SX, dimethylvinphos + SX, dimpropyridaz + SX, dinotefuran + SX, disodium octaborate + SX, disulfoton + SX, DNOC(2-methyl-4,6-dinitrophenol) + SX, doramectin + SX, dried leaves of Dryopteris filix-mas + SX, emamectin-benzoate + SX, empenthrin + SX, endosulfan + SX, EPN(O-ethyl O-(4-nitrophenyl) phenylphosphonothioate) + SX, epsilon-metofluthrin + SX, epsilon-momfluorothrin + SX, esfenvalerate + SX, ethiofencarb + SX, ethion + SX, ethiprole + SX, ethoprophos + SX, etofenprox + SX, etoxazole + SX, extract of Artemisia absinthium + SX, extract of Azadirachta indica + SX, extract of Cassia nigricans + SX, extract of clitoria ternatea + SX, extract of Symphytum officinale + SX, extract of Chenopodium ambrosioides + SX, extract of Tanacetum vulgare + SX, extract of Urtica dioica + SX, extract of Viscum album + SX, famphur + SX, fenamiphos + SX, fenazaquin + SX, fenbutatin oxide + SX, fenitrothion + SX, fenmezoditiaz + SX, fenobucarb + SX, fenoxycarb + SX, fenpropathrin + SX, fenpyroximate + SX, fenthion + SX, fenvalerate + SX, fipronil + SX, flometoquin + SX, flonicamid + SX, fluacrypyrim + SX, fluazaindolizine + SX, fluazuron + SX, flubendiamide + SX, fluchlordiniliprole + SX, flucycloxuron + SX, flucythrinate + SX, fluensulfone + SX, flufenoprox + SX, flufenoxuron + SX, flufiprole + SX, flumethrin + SX, flupentiofenox + SX, flupyradifurone + SX, flupyrimin + SX, flupyroxystrobin + SX, fluralaner + SX, fluvalinate + SX, fluxametamide + SX, formetanate + SX, fosthiazate + SX, furamethrin + SX, furathiocarb + SX, gamma-cyhalothrin + SX, GS-omega/kappa HXTX-Hvla peptide + SX, halfenprox + SX, halofenozide + SX, heptafluthrin + SX, heptenophos + SX, hexaflumuron + SX, hexythiazox + SX, potassium salt of hop beta acid + SX, hydramethylnon + SX, hydroprene + SX, imicyafos + SX, imidacloprid + SX, imidaclothiz + SX, imiprothrin + SX, indazapyroxamet + SX, indoxacarb + SX, isocycloseram + SX, isofenphos + SX, isoprocarb + SX, isopropyl-O-(methoxyaminothiophosphoryl) salicylate + SX, isoxathion + SX, ivermectin + SX, kadethrin + SX, kappa-tefluthrin + SX, kappa-bifenthrin + SX, kinoprene + SX, lambda-cyhalothrin + SX, ledprona + SX, lenoremycin + SX, lepimectin + SX, lime sulfur + SX, lotilaner + SX, lufenuron + SX, machine oil + SX, malathion + SX, mecarbam + SX, meperfluthrin + SX, metaflumizone + SX, metam + SX, methamidophos + SX, methidathion + SX, methiocarb + SX, methomyl + SX, methoprene + SX, methoxychlor + SX, methoxyfenozide + SX, methyl bromide + SX, metofluthrin + SX, metolcarb + SX, metoxadiazone + SX, mevinphos + SX, milbemectin + SX, milbemycin oxime + SX, mivorilaner + SX, modoflaner + SX, momfluorothrin + SX, monocrotophos + SX, moxidectin + SX, naled + SX, nicofluprole + SX, nicotine + SX, nicotine-sulfate + SX, nitenpyram + SX, novaluron + SX, noviflumuron + SX, oil of the seeds of Chenopodium anthelminticum + SX, omethoate + SX, oxamyl + SX, oxazosulfyl + SX, oxydemeton-methyl + SX, parathion + SX, parathion-methyl + SX, permethrin + SX, phenothrin + SX, phenthoate + SX, phorate + SX, phosalone + SX, phosmet + SX, phosphamidon + SX, phosphine + SX, phoxim + SX, pirimicarb + SX, pirimiphos-methyl + SX, prallethrin + SX, profenofos + SX, profluthrin + SX, propargite + SX, propetamphos + SX, propoxur + SX, propylene glycol alginate + SX, prothiofos + SX, pyflubumide + SX, pymetrozine + SX, pyraclofos + SX, pyrethrins + SX, pyridaben + SX, pyridalyl + SX, pyridaphenthion + SX, pyrifluquinazone + SX, pyrimidifen + SX, pyriminostrobin + SX, pyriprole + SX, pyriproxyfen + SX, quinalphos + SX, resmethrin + SX, rotenone + SX, ryanodine + SX, sarolaner + SX, selamectin + SX, sigma-cypermethrin + SX, silafluofen + SX, sodium borate + SX, sodium metaborate + SX, spidoxamat + SX, spinetoram + SX, spinosad + SX, spirobudifen + SX, spirodiclofen + SX, spiromesifen + SX, spiropidion + SX, spirotetramat + SX, sulfiflumin + SX, sulfluramid + SX, sulfotep + SX, sulfoxaflor + SX, sulfur + SX, sulfuryl fluoride + SX, tartar emetic + SX, tau-fluvalinate + SX, tebufenozide + SX, tebufenpyrad + SX, tebupirimfos + SX, teflubenzuron + SX, tefluthrin + SX, temephos + SX, terbufos + SX, terpene constituents of the extract of chenopodium ambrosioides near ambrosioides + SX, tetrachlorantraniliprole + SX, tetrachlorvinphos + SX, tetradifon + SX, tetramethrin + SX, tetramethylfluthrin + SX, tetraniliprole + SX, theta-cypermethrin + SX, thiacloprid + SX, thiamethoxam + SX, thiocyclam + SX, thiodicarb + SX, thiofanox + SX, thiometon + SX, thiosultap-disodium + SX, thiosultap-monosodium + SX, tigolaner + SX, tiorantraniliprole + SX, tioxazafen + SX, tolfenpyrad + SX, tralomethrin + SX, transfluthrin + SX, triazamate + SX, triazophos + SX, trichlorfon + SX, trifluenfuronate + SX, triflumezopyrim + SX, triflumuron + SX, trimethacarb + SX, tyclopyrazoflor + SX, umifoxolaner + SX, vamidothion + SX, wood extract of Quassia amara + SX, XMC (3,5-dimethylphenyl N-methylcarbamate) + SX, xylylcarb + SX, zeta-cypermethrin + SX, zinc phosphide + SX, 4-[5-(3,5-dichlorophenyl)-5-(trifluoromethyl)-4,5-dihydro-1,2-oxazol-3-yl]-2-methyl-N-(1-oxothietan-3-yl)benzamide (1241050-20-3) + SX, 3-methoxy-N-(5-{5-(trifluoromethyl)-5-[3-(trifluoromethyl)phenyl]-4,5-dihydro-1,2-oxazol-3-yl}indan-1-yl)propanamide (1118626-57-5) + SX, N-{4-chloro-3-[(1-cyanocyclopropyl)carbamoyl]phenyl}-1-methyl-4-(methanesulfonyl)-3-(1,1,2,2,2-pentafluoroethyl)-1H-pyrazole-3-carboxamide (1400768-21-9) + SX, N-[3-chloro-1-(pyridin-3-yl)-1H-pyrazol-4-yl]-2-(methanesulfonyl)propanamide (2396747-83-2) + SX, N-[4-chloro-2-(pyridin-3-yl)-1,3-thiazol-5-yl]-N-ethyl-3-(methanesulfonyl)propanamide + SX, 1,4-dimethyl-2-[2-(pyridin-3-yl)-2H-indazol-5-yl]-1,2,4-triazolidine-3,5-dione (2171099-09-3) + SX, 2-isopropyl-5-[(3,4,4-trifluoro-3-buten-1-yl)sulfonyl]-1,3,4-thiadiazole (2058052-95-0) + SX, N-({2-fluoro-4-[(2S,3S)-2-hydroxy-3-(3,4,5-trichlorophenyl)-3-(trifluoromethyl)pyrrolidin-1-yl]phenyl}methyl)cyclopropanecarboxamide + SX, 7-fluoro-N-[1-(methylsulfanyl)-2-methylpropan-2-yl]-2-(pyridin-3-yl)-2H-indazole-4-carboxamide + SX, 7-fluoro-N-[1-(methanesulfinyl)-2-methylpropan-2-yl]-2-(pyridin-3-yl)-2H-indazole-4-carboxamide + SX, 7-fluoro-N-[1-(methanesulfonyl)-2-methylpropan-2-yl]-2-(pyridin-3-yl)-2H-indazole-4-carboxamide + SX, N-[1-(difluoromethyl)cyclopropyl]-2-(pyridin-3-yl)-2H-indazole-4-carboxamide + SX, 2,9-dihydro-9-(methoxymethyl)-2-(pyridin-3-yl)-10H-pyrazolo[3,4-f]pyrido[2,3-b][1,4]oxazepin-10-one (2607927-97-7) + SX, BT crop protein Cry1Ab + SX, BT crop protein Cry1Ac + SX, BT crop protein Cry1Fa + SX, BT crop protein Cry1A.105 + SX, BT crop protein Cry2Ab + SX, BT crop protein Vip3A + SX, BT crop protein mCry3A + SX, BT crop protein Cry3Ab + SX, BT crop protein Cry3Bb + SX, BT crop protein Cry34Ab1/Cry35Ab1 + SX, Adoxophyes orana GV(granulovirus) strain BV-0001 + SX, Anticarsia gemmatalis MNPV(multiple nucleocapsid nucleopolyhedrovirus) + SX, Autographa californica MNPV + SX, Cydia pomonella GV strain V15 + SX, Cydia pomonella GV strain V22 + SX, Cryptophlebia leucotreta GV + SX, Dendrolimus punctatus cypovirus + SX, Helicoverpa armigera NPV(nucleopolyhedrovirus) strain BV-0003 + SX, Helicoverpa zea NPV + SX, Lymantria dispar NPV + SX, Mamestra brassicae NPV + SX, Mamestra configurata NPV + SX, Neodiprion abietis NPV + SX, Neodiprion lecontei NPV + SX, Neodiprion sertifer NPV + SX, Nosema locustae + SX, Orgyia pseudotsugata NPV + SX, Pieris rapae GV + SX, Plodia interpunctella GV + SX, Spodoptera exigua MNPV + SX, Spodoptera littoralis MNPV + SX, Spodoptera litura NPV + SX, Arthrobotrys dactyloides + SX, Bacillus firmus strain GB-126 + SX, Bacillus firmus strain I-1582 + SX, Bacillus firmus strain NCIM2637 + SX, Bacillus megaterium + SX, Bacillus sp. strain AQ175 + SX, Bacillus sp. strain AQ177 + SX, Bacillus sp. strain AQ178 + SX, Bacillus sphaericus strain 2362 serotype H5a5b + SX, Bacillus sphaericus strain ABTS1743 + SX, Bacillus thuringiensis strain AQ52 + SX, Bacillus thuringiensis strain BD#32 + SX, Bacillus thuringiensis strain CR-371 + SX, Bacillus thuringiensis subsp. Aizawai strain ABTS-1857 + SX, Bacillus thuringiensis subsp. Aizawai strain AM65-52 + SX, Bacillus thuringiensis subsp. Aizawai strain GC-91 + SX, Bacillus thuringiensis subsp. Aizawai strain NB200 + SX, Bacillus thuringiensis subsp. Aizawai Serotype strain H-7 + SX, Bacillus thuringiensis subsp. Kurstaki strain ABTS351 + SX, Bacillus thuringiensis subsp. Kurstaki strain BMP123 + SX, Bacillus thuringiensis subsp. Kurstaki strain CCT1306 + SX, Bacillus thuringiensis subsp. Kurstaki strain EG2348 + SX, Bacillus thuringiensis subsp. Kurstaki strain EG7841 + SX, Bacillus thuringiensis subsp. Kurstaki strain EVB113-19 + SX, Bacillus thuringiensis subsp. Kurstaki strain F810 + SX, Bacillus thuringiensis subsp. Kurstaki strain HD-1 + SX, Bacillus thuringiensis subsp. Kurstaki strain PB54 + SX, Bacillus thuringiensis subsp. Kurstaki strain SA-11 + SX, Bacillus thuringiensis subsp. Kurstaki strain SA-12 + SX, Bacillus thuringiensis subsp. Tenebriosis strain NB176 + SX, Bacillus thuringiensis subsp. Thuringiensis strain MPPL002 + SX, Bacillus thuringiensis subsp. morrisoni + SX, Bacillus thuringiensis var. colmeri + SX, Bacillus thuringiensis var. darmstadiensis strain 24-91 + SX, Bacillus thuringiensis var. dendrolimus + SX, Bacillus thuringiensis var. galleriae + SX, Bacillus thuringiensis var. israelensis strain BMP144 + SX, Bacillus thuringiensis var. israelensis serotype strain H-14 + SX, Bacillus thuringiensis var. japonensis strain buibui + SX, Bacillus thuringiensis var. san diego strain M-7 + SX, Bacillus thuringiensis var. 7216 + SX, Bacillus thuringiensis var. aegypti + SX, Bacillus thuringiensis var. T36 + SX, Beauveria bassiana strain ANT-03 + SX, Beauveria bassiana strain ATCC74040 + SX, Beauveria bassiana strain GHA + SX, Beauveria brongniartii + SX, Burkholderia rinojensis strain A396 + SX, Chromobacterium subtsugae strain PRAA4-1T + SX, Dactyllela ellipsospora + SX, Dectylaria thaumasia + SX, Hirsutella minnesotensis + SX, Hirsutella rhossiliensis + SX, Hirsutella thompsonii + SX, Lagenidium giganteum + SX, Lecanicillium lecanii strain KV01 + SX, Lecanicillium lecanii conidia of strain DAOM198499 + SX, Lecanicillium lecanii conidia of strain DAOM216596 + SX, Lecanicillium muscarium strain Ve6 + SX, Metarhizium anisopliae strain F52 + SX, Metarhizium anisopliae var. acridum + SX, Metarhizium anisopliae var. anisopliae BIPESCO 5/F52 + SX, Metarhizium flavoviride + SX, Monacrosporium phymatopagum + SX, Paecilomyces fumosoroseus Apopka strain 97 + SX, Paecilomyces lilacinus strain 251 + SX, Paecilomyces tenuipes strain T1 + SX, Paenibacillus popilliae + SX, Pasteuria nishizawae strain Pn1 + SX, Pasteuria penetrans + SX, Pasteuria usgae + SX, Pasteuria thornei + SX, Serratia entomophila + SX, Verticillium chlamydosporium + SX, Verticillium lecani strain NCIM1312 + SX, Wolbachia pipientis + SX.

Combination of the Present ingredient in the above Group (b) and the Present compound:
acibenzolar-S-methyl + SX, aldimorph + SX, ametoctradin + SX, aminopyrifen + SX, amisulbrom + SX, anilazine + SX, azaconazole + SX, azoxystrobin + SX, basic copper sulfate + SX, benalaxyl + SX, benalaxyl-M + SX, benodanil + SX, benomyl + SX, benthiavalicarb + SX, benthiavalicarb-isopropyl + SX, benzovindiflupyr + SX, binapacryl + SX, biphenyl + SX, bitertanol + SX, bixafen + SX, blasticidin-S + SX, Bordeaux mixture + SX, boscalid + SX, bromothalonil + SX, bromuconazole + SX, bupirimate + SX, captafol + SX, captan + SX, carbendazim + SX, carboxin + SX, carpropamid + SX, chinomethionat + SX, chitin + SX, chloroinconazide + SX, chloroneb + SX, chlorothalonil + SX, chlozolinate + SX, colletochlorin B + SX, copper(II) acetate + SX, copper(II) hydroxide + SX, copper oxychloride + SX, copper(II) sulfate + SX, coumoxystrobin + SX, cyazofamid + SX, cyflufenamid + SX, cymoxanil + SX, cyproconazole + SX, cyprodinil + SX, dichlobentiazox + SX, dichlofluanid + SX, diclocymet + SX, diclomezine + SX, dicloran + SX, diethofencarb + SX, difenoconazole + SX, diflumetorim + SX, dimethachlone + SX, dimethirimol + SX, dimethomorph + SX, dimoxystrobin + SX, diniconazole + SX, diniconazole-M + SX, dinocap + SX, dipotassium hydrogenphosphite + SX, dipymetitrone + SX, dithianon + SX, dodecylbenzenesulphonic acid bisethylenediamine copper(II) salt + SX, dodemorph + SX, dodine + SX, edifenphos + SX, enoxastrobin + SX, epoxiconazole + SX, etaconazole + SX, ethaboxam + SX, ethirimol + SX, etridiazole + SX, extract of Allium sativum + SX, extract of the cotyledons of lupine plantlets ("BLAD") + SX, extract of Equisetum arvense + SX, extract of Melaleuca alternifolia + SX, extract of Reynoutria sachalinensis + SX, extract of Tropaeolum majus + SX, famoxadone + SX, fenamidone + SX, fenaminstrobin + SX, fenarimol + SX, fenbuconazole + SX, fenfuram + SX, fenhexamid + SX, fenoxanil + SX, fenpiclonil + SX, fenpicoxamid + SX, fenpropidin + SX, fenpropimorph + SX, fenpyrazamine + SX, fentin acetate + SX, fentin chloride + SX, fentin hydroxide + SX, ferbam + SX, ferimzone + SX, florylpicoxamid + SX, fluazinam + SX, flubeneteram + SX, fludioxonil + SX, flufenoxadiazam + SX, flufenoxystrobin + SX, fluindapyr + SX, flumetylsulforim + SX, flumorph + SX, fluopicolide + SX, fluopyram + SX, fluopimomide + SX, fluoroimide + SX, fluoxapiprolin + SX, fluoxastrobin + SX, fluoxytioconazole + SX, fluquinconazole + SX, flusilazole + SX, flusulfamide + SX, flutianil + SX, flutolanil + SX, flutriafol + SX, fluxapyroxad + SX, folpet + SX, fosetyl + SX, fosetyl-aluminium + SX, fuberidazole + SX, furalaxyl + SX, furametpyr + SX, guazatine + SX, hexaconazole + SX, hymexazole + SX, imazalil + SX, imibenconazole + SX, iminoctadine + SX, iminoctadine triacetate + SX, inpyrfluxam + SX, iodocarb + SX, ipconazole + SX, ipfentrifluconazole + SX, ipflufenoquin + SX, iprobenfos + SX, iprodione + SX, iprovalicarb + SX, isofetamid + SX, isoflucypram + SX, isoprothiolane + SX, isopyrazam + SX, isotianil + SX, kasugamycin + SX, kresoxim-methyl + SX, laminarin + SX, leaves and bark of Quercus + SX, mancozeb + SX, mandestrobin + SX, mandipropamid + SX, maneb + SX, mefentrifluconazole + SX, mepanipyrim + SX, mepronil + SX, meptyldinocap + SX, metalaxyl + SX, metalaxyl-M + SX, metarylpicoxamid + SX, metconazole + SX, methasulfocarb + SX, metiram + SX, metominostrobin + SX, metrafenone + SX, metyltetraprole + SX, myclobutanil + SX, naftifine + SX, nuarimol + SX, octhilinone + SX, ofurace + SX, orysastrobin + SX, oxadixyl + SX, oxathiapiprolin + SX, oxine-copper + SX, oxolinic acid + SX, oxpoconazole + SX, oxpoconazole fumarate + SX, oxycarboxin + SX, oxytetracycline + SX, pefurazoate + SX, penconazole + SX, pencycuron + SX, penflufen + SX, penthiopyrad + SX, phenamacril + SX, phosphorous acid + SX, phthalide + SX, picarbutrazox + SX, picoxystrobin + SX, piperalin + SX, polyoxins + SX, potassium hydrogencarbonate + SX, potassium dihydrogenphosphite + SX, methyl 3-methoxy-2-((4-methyl-[1,1'-biphenyl]-3-yl)oxy)acrylate + SX, probenazole + SX, prochloraz + SX, procymidone + SX, propamidine + SX, propamocarb + SX, propiconazole + SX, propineb + SX, proquinazid + SX, prothiocarb + SX, prothioconazole + SX, pydiflumetofen + SX, pyraclostrobin + SX, pyrametostrobin + SX, pyraoxystrobin + SX, pyrapropoyne + SX, pyraziflumid + SX, pyrazophos + SX, pyribencarb + SX, pyributicarb + SX, pyridachlometyl + SX, pyrifenox + SX, pyrimethanil + SX, pyrimorph + SX, pyriofenone + SX, pyrisoxazole + SX, pyroquilon + SX, Quillaja extract + SX, quinconazole + SX, quinofumelin + SX, quinoxyfen + SX, quintozene + SX, Saponins of Chenopodium quinoa + SX, seboctylamine + SX, sedaxane + SX, silthiofam + SX, simeconazole + SX, sodium hydrogencarbonate + SX, spiroxamine + SX, streptomycin + SX, sulfur + SX, tebuconazole + SX, tebufloquin + SX, teclofthalam + SX, tecnazene + SX, terbinafine + SX, tetraconazole + SX, thiabendazole + SX, thifluzamide + SX, thiophanate + SX, thiophanate-methyl + SX, thiram + SX, thymol + SX, tiadinil + SX, tolclofos-methyl + SX, tolfenpyrad + SX, tolprocarb + SX, tolylfluanid + SX, triadimefon + SX, triadimenol + SX, triazoxide + SX, triclopyricarb + SX, tricyclazole + SX, tridemorph + SX, trifloxystrobin + SX, triflumizole + SX, triforine + SX, triticonazole + SX, validamycin + SX, valifenalate + SX, vinclozolin + SX, yellow mustard powder + SX, zinc thiazole + SX, zineb + SX, ziram + SX, zoxamide + SX, N'-[4-({3-[(4-chlorophenyl)methyl]-1,2,4-thiadiazol-5-yl}oxy)-2,5-dimethylphenyl]-N-ethyl-N-methylmethanimidamide (1202781-91-6) + SX, N'-{4-[(4,5-dichlorothiazol-2-yl)oxy]-2,5-dimethylphenyl}-N-ethyl-N-methylmethanimidamide (929908-57-6) + SX, N'-(2,5-dimethyl-4-phenoxyphenyl)-N-ethyl-N-methylmethanimidamide (1052688-31-9) + SX, N'-[5-chloro-4-(2-fluorophenoxy)-2-methylphenyl]-N-ethyl-N-methylmethanimidamide (2055589-28-9) + SX, N'-[2-chloro-4-(2-fluorophenoxy)-5-methylphenyl]-N-ethyl-N-methylmethanimidamide (2055756-21-1) + SX, N'-(2-chloro-4-phenoxy-5-methylphenyl)-N-ethyl-N-methylmethanimidamide (2062599-39-5) + SX, N'-[4-(1-hydroxy-1-phenyl-2,2,2-trifluoroethyl)-2-methyl-5-methoxyphenyl]-N-isopropyl-N-methylmethanimidamide (2101814-55-3) + SX, N'-[5-bromo-6-(1-methyl-2-propoxyethoxy)-2-methylpyridin-3-yl]-N-ethyl-N-methylmethanimidamide (1817828-69-5) + SX, 4-(2-bromo-4-fluorophenyl)-N-(2-chloro-6-fluorophenyl)-1,3-dimethyl-1H-pyrazol-5-amine (1362477-26-6) + SX, 2-[6-(3-fluoro-4-methoxyphenyl)-5-methylpyridin-2-yl]quinazoline (1257056-97-5) + SX, ethyl (2Z)-3-amino-2-cyano-3-phenylacrylate (39491-78-6) + SX, N-[(2-chlorothiazol-5-yl)methyl]-N-ethyl-6-methoxy-3-nitropyridin-2-amine (1446247-98-8) + SX, 5-(4-chlorobenzyl)-2-(chloromethyl)-2-methyl-1-(1H-1,2,4-triazol-1-ylmethyl)cyclopentan-1-ol (1394057-11-4) + SX, (1R, 2S, 5S)-5-(4-chlorobenzyl)-2-(chloromethyl)-2-methyl-1-(1H-1,2,4-triazol-1-ylmethyl)cyclopentan-1-ol (1801930-06-2) + SX, (1S, 2R, 5R)-5-(4-chlorobenzyl)-2-(chloromethyl)-2-methyl-1-(1H-1,2,4-triazol-1-ylmethyl)cyclopentan-1-ol (1801930-07-3) + SX, 2-(chloromethyl)-5-(4-fluorobenzyl)-2-methyl-1-(1H-1,2,4-triazol-1-ylmethyl)cyclopentan-1-ol (1394057-13-6) + SX, (1R, 2S, 5S)-2-(chloromethyl)-5-(4-fluorobenzyl)-2-methyl-1-(1H-1,2,4-triazol-1-ylmethyl)cyclopentan-1-ol (1801930-08-4) + SX, (1S, 2R, 5R)-2-(chloromethyl)-5-(4-fluorobenzyl)-2-methyl-1-(1H-1,2,4-triazol-1-ylmethyl)cyclopentan-1-ol (1801930-09-5) + SX, methyl 3-[(4-chlorophenyl)methyl]-2-hydroxy-1-methyl-2-(1H-1,2,4-triazol-1-ylmethyl)cyclopentan-1-carboxylate (1791398-02-1) + SX, 1-(2,4-difluorophenyl)-2-(1H-1,2,4-triazol-1-yl)-1-[1-(4-bromo-2,6-difluorophenoxy)cyclopropyl]ethanol (2019215-86-0) + SX, 1-(2,4-difluorophenyl)-2-(1H-1,2,4-triazol-1-yl)-1-[1-(4-chloro-2,6-difluorophenoxy)cyclopropyl]ethanol (2019215-84-8) + SX, 1-[2-(1-chlorocyclopropyl)-3-(2-fluorophenyl)-2-hydroxypropyl]-1H-imidazole-5-carbonitrile (2018316-13-5) + SX, 1-[2-(1-chlorocyclopropyl)-3-(2,3-difluorophenyl)-2-hydroxypropyl]-1H-imidazole-5-carbonitrile (2018317-25-2) + SX, 2-[6-(4-bromophenoxy)-2-(trifluoromethyl)pyridin-3-yl]-1-(1H-1,2,4-triazol-1-yl)propan-2-ol (2082661-43-4) + SX, 2-[6-(4-chlorophenoxy)-2-(trifluoromethyl)pyridin-3-yl]-1-(1H-1,2,4-triazol-1-yl)propan-2-ol (2082660-27-1) + SX, methyl ({2-methyl-5-[1-(4-methoxy-2-methylphenyl)-1H-pyrazol-3-yl]phenyl}methyl)carbamate (1605879-98-8) + SX, 2-(difluoromethyl)-N-[1,1,3-trimethyl-2,3-dihydro-1H-inden-4-yl]pyridine-3-carboxamide (1616239-21-4) + SX, 2-(difluoromethyl)-N-[3-ethyl-1,1-dimethyl-2,3-dihydro-1H-inden-4-yl]pyridine-3-carboxamide (1847460-02-9) + SX, 2-(difluoromethyl)-N-[3-propyl-1,1-dimethyl-2,3-dihydro-1H-inden-4-yl]pyridine-3-carboxamide (1847460-05-2) + SX, (2E,32)-5-{[1-(4-chlorophenyl)-1H-pyrazol-3-yl]oxy}-2-(methoxyimino)-N,3-dimethylpent-3-enamide (1445331-27-0) + SX, (2E,32)-5-{[1-(2,4-dichlorophenyl)-1H-pyrazol-3-yl]oxy}-2-(methoxyimino)-N,3-dimethylpent-3-enamide (1445331-54-3) + SX, 5-chloro-4-({2-[6-(4-chlorophenoxy)pyridin-3-yl]ethyl}amino)-6-methylpyrimidine (1605340-92-8) + SX, N-(1-benzyl-1,3-dimethylbutyl)-8-fluoroquinoline-3-carboxamide (2132414-04-9) + SX, N-(1-benzyl-3,3,3-trifluoro-1-methylpropyl)-8-fluoroquinoline-3-carboxamide (2132414-00-5) + SX, 4,4-dimethyl-2-({4-[5-(trifluoromethyl)-1,2,4-oxadiazol-3-yl]phenyl}methyl)isoxazolidin-3-one (2098918-25-1) + SX, 5,5-dimethyl-2-({4-[5-(trifluoromethyl)-1,2,4-oxadiazol-3-yl]phenyl}methyl)isoxazolidin-3-one (2098918-26-2) + SX, N-ethyl-2-methyl-N-({4-[5-(trifluoromethyl)-1,2,4-oxadiazol-3-yl]phenyl}methyl)propanamide + SX, N,2-dimethoxy-N-({4-[5-(trifluoromethyl)-1,2,4-oxadiazol-3-yl]phenyl}methyl)propanamide + SX, N-methoxy-N-({4-[5-(trifluoromethyl)-1,2,4-oxadiazol-3-yl]phenyl}methyl)cyclopropanecarboxamide + SX, N-methoxy-N'-methyl-N-({4-[5-(trifluoromethyl)-1,2,4-oxadiazol-3-yl]phenyl}methyl)urea + SX, N'-ethyl-N-methoxy-N-({4-[5-(trifluoromethyl)-1,2,4-oxadiazol-3-yl]phenyl}methyl)urea + SX, N,N'-dimethoxy-N-({4-[5-(trifluoromethyl)-1,2,4-oxadiazol-3-yl]phenyl}methyl)urea + SX, N-acetyl-2-(ethanesulfonyl)-N-[2-(methoxycarbonyl)-4-(trifluoromethoxy)phenyl]-4-(trifluoromethyl)benzamide (2043675-28-9) + SX, 3-(4-bromo-7-fluoroindol-1-yl)butan-2-yl N-[(3-hydroxy-4-methoxypyridin-2-yl)carbonyl]-L-alaninate + SX, 3-(7-bromoindol-1-yl)butan-2-yl N-[(3-hydroxy-4-methoxypyridin-2-yl)carbonyl]-L-alaninate + SX, 3-(7-bromo-4-fluoroindol-1-yl)butan-2-yl N-[(3-hydroxy-4-methoxypyridin-2-yl)carbonyl]-L-alaninate + SX, 3-(3,5-dichloropyridin-2-yl)butan-2-yl N-[(3-hydroxy-4-methoxypyridin-2-yl)carbonyl]-L-alaninate + SX, 3-(3,5-dichloropyridin-2-yl)butan-2-yl N-{[3-(acetoxymethoxy)-4-methoxypyridin-2-yl]carbonyl}-L-alaninate + SX, (1S)-1-[1-(naphthalen-1-yl)cyclopropyl]ethyl N-[(3-hydroxy-4-methoxypyridin-2-yl)carbonyl]-L-alaninate + SX, (1S)-1-[1-(naphthalen-1-yl)cyclopropyl]ethyl N-[(3-acetoxy-4-methoxypyridin-2-yl)carbonyl]-L-alaninate + SX, (1S)-1-[1-(naphthalen-1-yl)cyclopropyl]ethyl N-{[3-(acetoxymethoxy)-4-methoxypyridin-2-yl]carbonyl}-L-alaninate + SX, N-({4-[5-(trifluoromethyl)-1,2,4-oxadiazol-3-yl]phenyl}methyl)cyclopropanecarboxamide + SX, N-allyl-N-({4-[5-(trifluoromethyl)-1,2,4-oxadiazol-3-yl]phenyl}methyl)acetamide + SX, N-allyl-N-({4-[5-(trifluoromethyl)-1,2,4-oxadiazol-3-yl]phenyl}methyl)propanamide + SX, N-({4-[5-(trifluoromethyl)-1,2,4-oxadiazol-3-yl]phenyl}methyl)propanamide + SX, 3,3,3-trifluoro-N-({2-fluoro-4-[5-(trifluoromethyl)-1,2,4-oxadiazol-3-yl]phenyl}methyl)propanamide + SX, 3,3,3-trifluoro-N-({3-fluoro-4-[5-(trifluoromethyl)-1,2,4-oxadiazol-3-yl]phenyl}methyl)propanamide + SX, 3,3,3-trifluoro-N-({2,3-difluoro-4-[5-(trifluoromethyl)-1,2,4-oxadiazol-3-yl]phenyl}methyl)propanamide + SX, N-({2,3-difluoro-4-[5-(trifluoromethyl)-1,2,4-oxadiazol-3-yl]phenyl}methyl)butanamide + SX, N-methoxy-N-methyl-N'-({ 4-[5-(trifluoromethyl)-1,2,4-oxadiazol-3-yl]phenyl}methyl)urea + SX, N,N-diethyl-N'-({ 4-[5-(trifluoromethyl)-1,2,4-oxadiazol-3-yl]phenyl}methyl)urea + SX, N-methyl-N'-({ 4-[5-(trifluoromethyl)-1,2,4-oxadiazol-3-yl]phenyl}methyl)urea + SX, 1-({4-[5-(trifluoromethyl)-1,2,4-oxadiazol-3-yl]phenyl}methyl)pyrrolidin-2-one + SX, 1-({4-[5-(trifluoromethyl)-1,2,4-oxadiazol-3-yl]phenyl}methyl)piperidin-2-one + SX, 4-({4-[5-(trifluoromethyl)-1,2,4-oxadiazol-3-yl]phenyl}methyl)morpholin-3-one + SX, 2-({4-[5-(trifluoromethyl)-1,2,4-oxadiazol-3-yl]phenyl}methyl)isoxazolidin-3-one + SX, 3,3-dimethyl-1-({4-[5-(trifluoromethyl)-1,2,4-oxadiazol-3-yl]phenyl}methyl)piperidin-2-one + SX, 2-({4-[5-(trifluoromethyl)-1,2,4-oxadiazol-3-yl]phenyl}methyl)-1,2-oxazinan-3-one + SX, 1-({3-fluoro-4-[5-(trifluoromethyl)-1,2,4-oxadiazol-3-yl]phenyl}methyl)azepan-2-one + SX, 4,4-dimethyl-1-({4-[5-(trifluoromethyl)-1,2,4-oxadiazol-3-yl]phenyl}methyl)pyrrolidin-2-one + SX, 5-methyl-1-({4-[5-(trifluoromethyl)-1,2,4-oxadiazol-3-yl]phenyl}methyl)pyrrolidin-2-one + SX, ethyl 1-({4-[5-(trifluoromethyl)-1,2,4-oxadiazol-3-yl]phenyl}methyl)-1H-pyrazole-4-carboxylate + SX, N-methyl-1-({4-[5-(trifluoromethyl)-1,2,4-oxadiazol-3-yl]phenyl}methyl)-1H-pyrazole-4-carboxamide + SX, N-propyl-1-({4-[5-(trifluoromethyl)-1,2,4-oxadiazol-3-yl]phenyl}methyl)-1H-pyrazole-4-carboxamide + SX, N-methoxy-1-({4-[5-(trifluoromethyl)-1,2,4-oxadiazol-3-yl]phenyl}methyl)-1H-pyrazole-4-carboxamide + SX, N-methoxy-N-methyl-1-({4-[5-(trifluoromethyl)-1,2,4-oxadiazol-3-yl]phenyl}methyl)-1H-pyrazole-4-carboxamide + SX, N,N-dimethyl-1-({4-[5-(trifluoromethyl)-1,2,4-oxadiazol-3-yl]phenyl}methyl)-1H-1,2,4-triazol-3-amine + SX, N-methyl-4-[5-(trifluoromethyl)-1,2,4-oxadiazol-3-yl]benzamide + SX, methyl 2-[2-chloro-4-(4-chlorophenoxy)phenyl]-2-hydroxy-3-(1H-1,2,4-triazol-1-yl)propanoate + SX, ethyl 2-[2-chloro-4-(4-chlorophenoxy)phenyl]-2-hydroxy-3-(1H-1,2,4-triazol-1-yl)propanoate + SX, methyl 2-[2-(trifluoromethyl)-4-(4-chlorophenoxy)phenyl]-2-hydroxy-3-(1H-1,2,4-triazol-1-yl)propanoate + SX, 1-(2,3-dimethylpyridin-5-yl)-4,4-difluoro-3,3-dimethyl-3,4-dihydroisoquinoline + SX, 1-[2-(difluoromethyl)-3-methylpyridin-5-yl]-4,4-difluoro-3,3-dimethyl-3,4-dihydroisoquinoline + SX, 2,2-difluoro-N-[6-({[1-(1-methyl-1H-tetrazol-5-yl)benzimidazol-2-yl]oxy}methyl)pyridin-2-yl]-2-phenoxyacetamide + SX, 1-[2-(1-chlorocyclopropyl)-3-(3-chloro-2-fluorophenyl)-2-hydroxypropyl]-1H-imidazole-5-carbonitrile + SX, ethyl 1-[(4-{[2-(trifluoromethyl)-1,3-dioxolan-2-yl]methoxy}phenyl)methyl]-1H-pyrazole-4-carboxylate + SX, ethyl 1-[(4-{[(1Z)-2-ethoxy-3,3,3-trifluoro-1-propen-1-yl]oxy}phenyl)methyl]-1H-pyrazole-4-carboxylate + SX, 6-chloro-3-(3-cyclopropyl-2-fluorophenoxy)-N-[2-(2,4-dimethylphenyl)-2,2-difluoroethyl]-5-methylpyridazine-4-carboxamide + SX, 6-chloro-3-(3-cyclopropyl-2-fluorophenoxy)-N-[2-(3,4-dimethylphenyl)-2,2-difluoroethyl]-5-methylpyridazine-4-carboxamide + SX, 6-chloro-N-[2-(2-chloro-4-methylphenyl)-2,2-difluoroethyl]-3-(3-cyclopropyl-2-fluorophenoxy)-5-methylpyridazine-4-carboxamide + SX, 2-[cyano(2,6-difluoropyridin-4-yl)amino]-N-(2,2-dimethylcyclobutyl)-5-methylthiazole-4-carboxamide + SX, 2-[cyano(2,6-difluoropyridin-4-yl)amino]-N-(spiro[3.4]octan-1-yl)-5-methylthiazole-4-carboxamide + SX, 2-[cyano(2,6-difluoropyridin-4-yl)amino]-N-hexyl-5-methylthiazole-4-carboxamide + SX, 2-[acetyl(2,6-difluoropyridin-4-yl)amino]-N-(2,2-dimethylcyclobutyl)-5-methylthiazole-4-carboxamide + SX, 2-[(2-methoxyacetyl)(2,6-difluoropyridin-4-yl)amino]-N-(2,2-dimethylcyclobutyl)-5-methylthiazole-4-carboxamide + SX, 2-[(2-methylpropanoyl)(2,6-difluoropyridin-4-yl)amino]-N-(2,2-dimethylcyclobutyl)-5-methylthiazole-4-carboxamide + SX, 2-[(2,6-difluoropyridin-4-yl)amino]-N-(2,2-dimethylcyclobutyl)-5-methylthiazole-4-carboxamide + SX, 2-{[(oxetan-3-yl)carbonyl](2,6-difluoropyridin-4-yl)amino}-N-(2,2-dimethylcyclobutyl)-5-methylthiazole-4-carboxamide + SX, 2-{[(oxolan-3-yl)carbonyl](2,6-difluoropyridin-4-yl)amino}-N-(2,2-dimethylcyclobutyl)-5-methylthiazole-4-carboxamide + SX, 2-{[(oxan-4-yl)carbonyl](2,6-difluoropyridin-4-yl)amino}-N-(2,2-dimethylcyclobutyl)-5-methylthiazole-4-carboxamide + SX, 5-[5-(difluoromethyl)-1,3,4-oxadiazol-2-yl]-N-[1-(2-fluorophenyl)ethyl]pyrimidin-2-amine + SX, 5-[5-(difluoromethyl)-1,3,4-oxadiazol-2-yl]-N-[1-(2,6-difluorophenyl)ethyl]pyrimidin-2-amine + SX, 5-[5-(difluoromethyl)-1,3,4-oxadiazol-2-yl]-N-[1-(3,5-difluorophenyl)ethyl]pyrimidin-2-amine + SX, 5-[5-(difluoromethyl)-1,3,4-oxadiazol-2-yl]-N-[1-(6-chloropyridin-3-yl)ethyl]pyrimidin-2-amine + SX, 5-[5-(difluoromethyl)-1,3,4-oxadiazol-2-yl]-N-[1-(2-fluorophenyl)cyclopropyl]pyrimidin-2-amine + SX, 5-[5-(difluoromethyl)-1,3,4-oxadiazol-2-yl]-N-[1-(2,6-difluorophenyl)cyclopropyl]pyrimidin-2-amine + SX, 5-[5-(difluoromethyl)-1,3,4-oxadiazol-2-yl]-N-[1-(2-fluoro-3-methoxyphenyl)cyclopropyl]pyrimidin-2-amine + SX, 5-[5-(difluoromethyl)-1,3,4-oxadiazol-2-yl]-2-{[1-(2,6-difluorophenyl)cyclopropyl]oxy}pyrimidine + SX, 3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, (5S)-3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, 3-[3-(3-chloro-2-fluorophenoxy)-6-methylpyridazin-4-yl]-5-[(4-bromo-2-methylphenyl)methyl]-5,6-dihydro-4H-1,2,4-oxadiazine + SX, 3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5S)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5R)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2-methylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2,2-dimethylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(5-fluoro-2-methoxyphenyl)-2-(2-cyanoethoxy)ethyl]-5-[1-(isopropoxyimino)ethyl]-2,6-dioxo-3,6-dihydropyrimidin-1(2H)-yl}propan-2-yl)-2-methylpropanamide + SX, methyl ({5-[1-(2,6-difluoro-4-isopropylphenyl)-1H-pyrazol-3-yl]-2-methylphenyl}methyl)carbamate + SX, methyl ({5-[1-(2,6-difluoro-4-cyclopropylphenyl)-1H-pyrazol-3-yl]-2-methylphenyl}methyl)carbamate + SX, methyl ({5-[1-(2,6-difluoro-4-methoxyphenyl)-1H-pyrazol-3-yl]-2-methylphenyl}methyl)carbamate + SX, methyl (Z)-2-(5-cyclopentyl-2-methylphenoxy)-3-methoxyprop-2-enoate + SX, methyl (Z)-2-(5-cyclohexyl-2-methylphenoxy)-3-methoxyprop-2-enoate + SX, methyl (Z)-2-[(3-isopropyl-1H-pyrazol-1-yl)-2-methylphenoxy]-3-methoxyprop-2-enoate + SX, 1-(4,5-dimethyl-1H-benzimidazol-1-yl)-4,4-difluoro-3,3-dimethyl-3,4-dihydroisoquinoline + SX, 1-(4,5-dimethyl-1H-benzimidazol-1-yl)-4,4,5-trifluoro-3,3-dimethyl-3,4-dihydroisoquinoline + SX, 1-(pyrazolo[1,5-a]pyridin-3-yl)-4,4-difluoro-3,3-dimethyl-3,4-dihydroisoquinoline + SX, 1-(6,7-dimethylpyrazolo[1,5-a]pyridin-3-yl)-6-fluoro-3,3-dimethylisoquinolin-4(3H)-one + SX, Agrobacterium radiobactor strain K1026 + SX, Agrobacterium radiobactor strain K84 + SX, Bacillus amyloliquefaciens strain PTA-4838 (Aveo (registered trademark) EZ Nematicide) + SX, Bacillus amyloliquefaciens strain AT332 + SX, Bacillus amyloliquefaciens strain B3 + SX, Bacillus amyloliquefaciens strain D747 + SX, Bacillus amyloliquefaciens strain DB101 + SX, Bacillus amyloliquefaciens strain DB102 + SX, Bacillus amyloliquefaciens strain GB03 + SX, Bacillus amyloliquefaciens strain FZB24 + SX, Bacillus amyloliquefaciens strain FZB42 + SX, Bacillus amyloliquefaciens strain IN937a + SX, Bacillus amyloliquefaciens strain MBI600 + SX, Bacillus amyloliquefaciens strain QST713 + SX, Bacillus amyloliquefaciens isolate strain B246 + SX, Bacillus amyloliquefaciens strain F727 + SX, Bacillus amyloliquefaciens subsp. plantarum strain D747 + SX, Bacillus licheniformis strain HB-2 + SX, Bacillus licheniformis strain SB3086 + SX, Bacillus pumilus strain AQ717 + SX, Bacillus pumilus strain BUF-33 + SX, Bacillus pumilus strain GB34 + SX, Bacillus pumilus strain QST2808 + SX, Bacillus simplex strain CGF2856 + SX, Bacillus subtilis strain AQ153 + SX, Bacillus subtilis strain AQ743 + SX, Bacillus subtilis strain BU1814 + SX, Bacillus subtilis strain D747 + SX, Bacillus subtilis strain DB101 + SX, Bacillus subtilis strain FZB24 + SX, Bacillus subtilis strain GB03 + SX, Bacillus subtilis strain HAI0404 + SX, Bacillus subtilis strain IAB/BS03 + SX, Bacillus subtilis strain MBI600 + SX, Bacillus subtilis strain QST30002/AQ30002 + SX, Bacillus subtilis strain QST30004/AQ30004 + SX, Bacillus subtilis strain QST713 + SX, Bacillus subtilis strain QST714 + SX, Bacillus subtilis var. Amyloliquefaciens strain FZB24 + SX, Bacillus subtilis strain Y1336 + SX, Burkholderia cepacia + SX, Burkholderia cepacia type Wisconsin strain J82 + SX, Burkholderia cepacia type Wisconsin strain M54 + SX, Candida oleophila strain O + SX, Candida saitoana + SX, Chaetomium cupreum + SX, Clonostachys rosea + SX, Coniothyrium minitans strain CGMCC8325 + SX, Coniothyrium minitans strain CON/M/91-8 + SX, cryptococcus albidus + SX, Erwinia carotovora subsp. carotovora strain CGE234M403 + SX, Fusarium oxysporum strain Fo47 + SX, Gliocladium catenulatum strain J1446 + SX, Paenibacillus polymyxa strain AC-1 + SX, Paenibacillus polymyxa strain BS-0105 + SX, Pantoea agglomerans strain E325 + SX, Phlebiopsis gigantea strain VRA1992 + SX, Pseudomonas aureofaciens strain TX-1 + SX, Pseudomonas chlororaphis strain 63-28 + SX, Pseudomonas chlororaphis strain AFS009 + SX, Pseudomonas chlororaphis strain MA342 + SX, Pseudomonas fluorescens strain 1629RS + SX, Pseudomonas fluorescens strain A506 + SX, Pseudomonas fluorescens strain CL145A + SX, Pseudomonas fluorescens strain G7090 + SX, Pseudomonas sp. strain CAB-02 + SX, Pseudomonas syringae strain 742RS + SX, Pseudomonas syringae strain MA-4 + SX, Pseudozyma flocculosa strain PF-A22UL + SX, Pseudomonas rhodesiae strain HAI-0804 + SX, Pythium oligandrum strain DV74 + SX, Pythium oligandrum strain M1 + SX, Streptomyces griseoviridis strain K61 + SX, Streptomyces lydicus strain WYCD108US + SX, Streptomyces lydicus strain WYEC108 + SX, Talaromyces flavus strain SAY-Y-94-01 + SX, Talaromyces flavus strain V117b + SX, Trichoderma asperellum strain ICC012 + SX, Trichoderma asperellum SKT-1 + SX, Trichoderma asperellum strain T25 + SX, Trichoderma asperellum strain T34 + SX, Trichoderma asperellum strain TV1 + SX, Trichoderma atroviride strain CNCM 1-1237 + SX, Trichoderma atroviride strain LC52 + SX, Trichoderma atroviride strain IMI 206040 + SX, Trichoderma atroviride strain SC1 + SX, Trichoderma atroviride strain SKT-1 + SX, Trichoderma atroviride strain T11 + SX, Trichoderma gamsii strain ICC080 + SX, Trichoderma harzianum strain 21 + SX, Trichoderma harzianum strain DB104 + SX, Trichoderma harzianum strain DSM 14944 + SX, Trichoderma harzianum strain ESALQ-1303 + SX, Trichoderma harzianum strain ESALQ-1306 + SX, Trichoderma harzianum strain IIHR-Th-2 + SX, Trichoderma harzianum strain ITEM908 + SX, Trichoderma harzianum strain kd + SX, Trichoderma harzianum strain MO1 + SX, Trichoderma harzianum strain SF + SX, Trichoderma harzianum strain T22 + SX, Trichoderma harzianum strain T39 + SX, Trichoderma harzianum strain T78 + SX, Trichoderma harzianum strain TH35 + SX, Trichoderma polysporum strain IMI206039 + SX, trichoderma stromaticum + SX, Trichoderma virens strain G-41 + SX, Trichoderma virens strain GL-21 + SX, Trichoderma viride + SX, Variovorax paradoxus strain CGF4526 + SX, Harpin protein + SX.

Combination of the Present ingredient in the above Group (c) and the Present compound:
1-methylcyclopropene + SX, 1,3-diphenylurea + SX, 2,3,5-triiodobenzoic acid + SX, IAA ((1H-indol-3-yl)acetic acid) + SX, IBA (4-(1H-indol-3-yl)butyric acid) + SX, MCPA (2-(4-chloro-2-methylphenoxy)acetic acid) + SX, MCPB (4-(4-chloro-2-methylphenoxy)butyric acid) + SX, 4-CPA (4-chlorophenoxyacetic acid) + SX, 5-aminolevulinic acid hydrochloride + SX, 6-benzylaminopurine + SX, abscisic acid + SX, AVG (aminoethoxyvinylglycine) + SX, anisiflupurin + SX, ancymidol + SX, butralin + SX, calcium carbonate + SX, calcium chloride + SX, calcium formate + SX, calcium peroxide + SX, calcium polysulfide + SX, calcium sulfate + SX, chlormequat-chloride + SX, chlorpropham + SX, choline chloride + SX, cloprop + SX, cyanamide + SX, cyclanilide + SX, daminozide + SX, decan-1-ol + SX, dichlorprop + SX, dikegulac + SX, dimethipin + SX, diquat + SX, ethephon + SX, ethychlozate + SX, flumetralin + SX, flurprimidol + SX, forchlorfenuron + SX, formononetin + SX, Gibberellin A + SX, Gibberellin A3 + SX, inabenfide + SX, Kinetin + SX, lipochitooligosaccharide SP104 + SX, maleic hydrazide + SX, mefluidide + SX, mepiquat-chloride + SX, oxidized glutathione + SX, paclobutrazol + SX, pendimethalin + SX, prohexadione-calcium + SX, prohydrojasmon + SX, pyraflufen-ethyl + SX, sintofen + SX, sodium 1-naphthaleneacetate + SX, sodium cyanate + SX, thidiazuron + SX, triapenthenol + SX, tribufos + SX, trinexapac-ethyl + SX, uniconazole-P + SX, 2-(naphthalen-1-yl)acetamide + SX, [4-oxo-4-(2-phenylethyl)amino]butylate + SX, methyl 5-(trifluoromethyl)benzo[b]thiophene-2-carboxylate + SX, 3-[(6-chloro-4-phenylquinazolin-2-yl)amino]propan-1-ol + SX, Claroideoglomus etunicatum + SX, Claroideoglomus claroideum + SX, Funneliformis mosseae + SX, Gigaspora margarita + SX, Gigaspora rosea + SX, Glomus aggregatum + SX, Glomus deserticola + SX, Glomus monosporum + SX, Paraglomus brasillianum + SX, Rhizophagus clarus + SX, Rhizophagus intraradices RTI-801 + SX, Rhizophagus irregularis DAOM 197198 + SX, Azorhizobium caulinodans + SX, Azospirillum amazonense + SX, Azospirillum brasilense XOH + SX, Azospirillum brasilense Ab-V5 + SX, Azospirillum brasilense Ab-V6 + SX, Azospirillum caulinodans + SX, Azospirillum halopraeferens + SX, Azospirillum irakense + SX, Azospirillum lipoferum + SX, Bradyrhizobium elkanii SEMIA 587 + SX, Bradyrhizobium elkanii SEMIA 5019 + SX, Bradyrhizobium japonicum TA-11 + SX, Bradyrhizobium japonicum USDA 110 + SX, Bradyrhizobium liaoningense + SX, Bradyrhizobium lupini + SX, Delftia acidovorans RAY209 + SX, Mesorhizobium ciceri + SX, Mesorhizobium huakii + SX, Mesorhizobium loti + SX, Rhizobium etli + SX, Rhizobium galegae + SX, Rhizobium leguminosarum bv. Phaseoli + SX, Rhizobium leguminosarum bv. Trifolii + SX, Rhizobium leguminosarum bv. Viciae + SX, Rhizobium trifolii + SX, Rhizobium tropici + SX, Sinorhizobium fredii + SX, Sinorhizobium meliloti + SX, Zucchini Yellow Mosaik Virus weak strain + SX.

Combination of the Present ingredient in the above Group (d) and the Present compound:
anthraquinone + SX, deet + SX, icaridin + SX.

The ratio of the Present compound to the Present ingredient includes, but not limited thereto, as a ratio by weight (the Present compound : the Present ingredient) 1,000 : 1 to 1 : 1,000, 500 : 1 to 1 : 500, 100 : 1 to 1 : 100, 50 : 1, 20 : 1, 10 : 1, 9 : 1, 8 : 1, 7 : 1, 6 : 1, 5 : 1, 4 : 1, 3 : 1, 2 : 1, 1 : 1, 1 : 2, 1 : 3, 1 : 4, 1 : 5, 1 : 6, 1 : 7, 1 : 8, 1 : 9, 1 : 10, 1 : 20, 1 : 50, and the others.

The Present compound can control plant diseases caused by plant pathogenic microorganisms such as fungi, Oomycete, Phytomyxea, and bacteria. Examples of the fungi include Ascomycota, Basidiomycota, Blastocladiomycota, Chytridiomycota, Mucoromycota, and Olpidiomycota. Specific examples thereof include the followings. The scientific name of plant pathogenic microorganism which causes each disease is shown in parentheses.

Rice diseases:
   blast (Pyricularia oryzae), brown spot (Cochliobolus miyabeanus), sheath blight (Rhizoctonia solani), bakanae disease (Gibberella fujikuroi), downy mildew (Sclerophthora macrospora), false blast and head blight (Epicoccum nigrum), seedling blight (Trichoderma viride and Rhizopus oryzae), pseudo sheath blight (Waitea circinate, Ceratobasidium setariae, and Thanatephorus cucumeris), Rice false smut (Ustilaginoidea virens), and Kernel smut (Tilletia horrida and Tilletia barclayana);
Wheat diseases:
   powdery mildew (Blumeria graminis), fusarium blight (Fusarium graminearum, Fusarium avenaceum, Fusarium culmorum, and Microdochium nivale), stripe rust (Puccinia striiformis), stem rust (Puccinia graminis), leaf rust (Puccinia recondita), snow mould (Microdochium nivale and Microdochium majus), typhula snow blight (Typhula incarnata and Typhula ishikariensis), Sclerotinia snow blight (Sclerotinia borealis), Browning root rot (Pythium spp.), loose smut (Ustilago tritici), stinking smut (Tilletia caries and Tilletia controversa), eyespot (Pseudocercosporella herpotrichoides), leaf blotch (Septoria tritici), glume blotch (Stagonospora nodorum), tan spot (Pyrenophora tritici-repentis), rhizoctonia seeding blight (Rhizoctonia solani), take-all disease (Gaeumannomyces graminis), and blast (Pyricularia graminis-tritici);
Barley diseases:
   powdery mildew (Blumeria graminis), fusarium head blight (Fusarium graminearum, Fusarium avenaceum, Fusarium culmorum, and Microdochium nivale), stripe rust (Puccinia striiformis), stem rust (Puccinia graminis), dwarf leaf rust (Puccinia hordei), loose smut (Ustilago nuda), scald (Rhynchosporium secalis), net blotch (Pyrenophora teres), spot blotch (Cochliobolus sativus), stripe (Pyrenophora graminea), Ramularia disease (Ramularia collo-cygni), and rhizoctonia seeding blight (Rhizoctonia solani);
Corn diseases:
   rust (Puccinia sorghi), southern rust (Puccinia polysora), northern leaf blight (Setosphaeria turcica), tropical rust (Physopella zeae), southern leaf blight (Cochliobolus heterostrophus), anthracnose (Colletotrichum graminicola), gray leaf spot (Cercospora zeae-maydis), eyespot (Kabatiella zeae), phaeosphaeria leaf spot (Phaeosphaeria maydis), diplodia rot (Stenocarpella maydis and Stenocarpella macrospora), stalk rot (Fusarium graminearum, Fusarium verticilioides, and Colletotrichum graminicola), smut (Ustilago maydis), Physoderma brown spot and Physoderma stalk rot (Physoderma maydis), and tar spot disease (Phyllachora maydis);
Cotton diseases:
   anthracnose (Colletotrichum gossypii), grey mildew (Ramularia areola), alternaria leaf spot (Alternaria macrospora and Alternaria gossypii), and black root rot (Thielaviopsis basicola);
Coffee diseases:
   rust (Hemileia vastatrix) and leaf spot (Cercospora coffeicola);
Rape seed diseases:
   sclerotinia rot (Sclerotinia sclerotiorum), gray leaf spot (Alternaria brassicae), root rot (Phoma lingam), and light leaf spot (Pyrenopeziza brassicae);
Sugar cane diseases:
   rust (Puccinia melanocephela and Puccinia kuehnii), and smut (Ustilago scitaminea);
Sunflower diseases:
   rust (Puccinia helianthi) and downy mildew (Plasmopara halstedii) ;
Citrus diseases:
   melanose (Diaporthe citri), scab (Elsinoe fawcetti), green mold (Penicillium digitatum), blue mold (Penicillium italicum), Phytophthora rot (Phytophthora parasitica and Phytophthora citrophthora), and aspergillus rot (Aspergillus niger) ;
Apple diseases:
   blossom blight (Monilinia mali), valsa canker (Valsa ceratosperma), powdery mildew (Podosphaera leucotricha), alternaria leaf spot (Alternaria alternata apple pathotype), scab (Venturia inaequalis), bitter rot (Glomerella cingulata and Colletotrichum acutatum), blotch (Diplocarpon mali), ring rot (Botryosphaeria berengeriana), crown rot (Phytophtora cactorum), and rust (Gymnosporangium juniperi-virginianae and Gymnosporangium yamadae);
Pear diseases:
   scab (Venturia nashicola and Venturia pirina), black spot (Alternaria alternata Japanese pear pathotype), and rust (Gymnosporangium haraeanum);
Peach diseases:
   brown rot (Monilinia fructicola), scab (Cladosporium carpophilum), Phomopsis rot (Phomopsis sp.), leaf curl (Taphrina deformans), and powdery mildew (Podosphaera leucotricha);
Grapes diseases:
   anthracnose (Elsinoe ampelina), ripe rot (Glomerella cingulata and Colletotrichum acutatum), powdery mildew (Uncinula necator), rust (Neophysopella sp.), black rot (Guignardia bidwellii), downy mildew (Plasmopara viticola), leaf blight (Pseudocercospora vitis), and white rot (Coniella castaneicola);
Japanese persimmon diseases:
   anthracnose (Gloeosporium kaki and Colletotrichum acutatum), leaf spot (Cercospora kaki and Mycosphaerella nawae), and powdery mildew (Phyllactinia kakicola);
Fig disease:
   rust (Phakopsora nishidana);
Diseases of gourd family:
   anthracnose (Colletotrichum lagenarium), powdery mildew (Sphaerotheca fuliginea), gummy stem blight (Didymella bryoniae), Corynespora leaf spot (Corynespora cassiicola), fusarium wilt (Fusarium oxysporum), downy mildew (Pseudoperonospora cubensis), phytophthora rot (Phytophthora capsici), damping-off (Pythium sp.), and Black root rot (Phomopsis sp.);
Tomato diseases:
   early blight (Alternaria solani), leaf mold (Cladosporium fulvum), Cercospora leaf mold (Pseudocercospora fuligena), late blight (Phytophthora infestans), and powdery mildew (Leveillula taurica);
Eggplant diseases:
   brown spot (Phomopsis vexans), powdery mildew (Erysiphe cichoracearum), black blight (Corynespora melongenae), leaf mold (Mycovellosiella nattrassii), and brown leaf spot (Thanatephorus cucumeris);
Cruciferous vegetables diseases:
   alternaria leaf spot (Alternaria japonica), white spot (Cercosporella brassicae), clubroot (Plasmodiophora brassicae), downy mildew (Peronospora parasitica), white rust (Albugo candida), and Alternaria sooty spot (Alternaria brassicicola);
Welsh onion diseases:
   rust (Puccinia allii), black spot (Alternaria porri), white rot (Sclerotium cepivorum), leaf blight (Botrytis squamosa), leaf spot (Stemphylium vesicarium and Stemphylium botryosum), southern blight (Sclerotium rolfsii), leaf blight (Botrytis squamosa);
Soybean diseases:
   purple stain (Cercospora kikuchii), sphaceloma scab (Elsinoe glycines), pod and stem blight (Diaporthe phaseolorum var. sojae), rust (Phakopsora pachyrhizi), target spot (Corynespora cassiicola), anthracnose (Colletotrichum glycines and Colletotrichum truncatum), Rhizoctonia rot (Rhizoctonia solani), septoria brown spot (Septoria glycines), Cercospora leaf spot (Cercospora sojina), stem rot (Sclerotinia sclerotiorum), powdery mildew (Microsphaera diffusa), phytophthora stem and root rot (Phytophthora sojae), downy mildew (Peronospora manshurica), sudden death syndrome (Fusarium virguliforme), red crown rot (Calonectria ilicicola), and Diaporthe/Phomopsis complex (Diaporthe longicolla, Diaporthe phaseolorum, and Phomopsis longicolla);
Kidney bean diseases:
   stem rot (Sclerotinia sclerotiorum), rust (Uromyces appendiculatus), angular leaf spot (Phaeoisariopsis griseola), anthracnose (Colletotrichum lindemuthianum), and Fusarium root-rot (Fusarium solani);
Peanut diseases:
   leaf spot (Cercospora personata), brown leaf spot (Cercospora arachidicola), southern blight (Sclerotium rolfsii), and Cylindrocladium black rot (Calonectria ilicicola);
Garden pea diseases:
   powdery mildew (Erysiphe pisi) and root rot (Fusarium solani);
Potato diseases:
   early blight (Alternaria solani), late blight (Phytophthora infestans), Pink rot (Phytophthora erythroseptica), powdery scab (Spongospora subterranea f. sp. subterranea), verticillium wilt (Verticillium albo-atrum, Verticillium dahliae, and Verticillium nigrescens), dry rot (Fusarium solani), and potato wart (Synchytrium endobioticum);
Strawberry diseases:
   powdery mildew (Sphaerotheca humuli) and crown rot (Glomerella cingulata and Colletotrichum acutatum);
Tea diseases:
   net blister blight (Exobasidium reticulatum), white scab (Elsinoe leucospila), gray blight (Pestalotiopsis sp.), and anthracnose (Colletotrichum theae-sinensis);
Tobacco diseases:
   brown spot (Alternaria longipes), anthracnose (Colletotrichum tabacum), blue mold (Peronospora tabacina), and black shank (Phytophthora nicotianae);
Sugar beet diseases:
   cercospora leaf spot (Cercospora beticola), leaf blight (Thanatephorus cucumeris), root rot (Thanatephorus cucumeris), aphanomyces root rot (Aphanomyces cochlioides), and rust (Uromyces betae);
Rose diseases:
   black spot (Diplocarpon rosae) and powdery mildew (Sphaerotheca pannosa);
Chrysanthemum diseases:
   leaf blight (Septoria chrysanthemi-indici) and white rust (Puccinia horiana);
Onion diseases:
   botrytis leaf blight (Botrytis cinerea, Botrytis byssoidea, and Botrytis squamosa), gray-mold neck rot (Botrytis allii), and small sclerotial neck rot (Botrytis squamosa);
Various crops diseases:
   Botrytis rot (Botrytis cinerea), sclerotinia rot (Sclerotinia sclerotiorum), white rot (Sclerotium cepivorum), southern blight (Sclerotium rolfsii), and seedling blight (Pythium aphanidermatum, Pythium irregulare, and Pythium ultimum);
Japanese radish disease:
   alternaria leaf spot (Alternaria brassicicola);
Sweet potato diseases:
   stem rot (Fusarium oxysporum f. sp. batatas), foot rot (Diaporthe destruens), and black rot (Ceratocystis fimbriata);
Turfgrass diseases:
   dollar spot (Sclerotinia homoeocarpa), brown patch and large patch (Rhizoctonia solani), pythium blight (Pythium aphanidermatum), and anthracnose (Colletotrichum caudatum);
Banana disease:
   Sigatoka disease (Mycosphaerella fijiensis and Mycosphaerella musicola);
Lentils disease:
   ascochyta blight (Ascochyta lentis);
Chickpea disease:
   ascochyta blight (Ascochyta rabiei);
Green pepper diseases:
   anthracnose (Colletotrichum scovillei), powdery mildew (Leveillula taurica), and southern blight (Sclerotium rolfsii);
Mango disease:
   anthracnose (Colletotrichum acutatum);
ocarina disease:
   foot rot (Diaporthe destruens);
Fruit trees diseases:
   white root rot (Rosellinia necatrix) and violet root rot (Helicobasidium mompa);
Postharvest disease of fruits (for example, apple and pear) :
   Mucor rot diseases (Mucor piriformis);
Seed diseases or diseases in the early stages of the growth of various plants caused by Aspergillus spp., Penicillium spp., Fusarium spp., Gibberella spp., Tricoderma spp., Thielaviopsis spp., Rhizopus spp., Mucor spp., Corticium spp., Phoma spp., Rhizoctonia spp. or Diplodia spp., or the like;
Viral diseases:
   Lettuce big-vein disease transmitted by Olpidium brassicae, and viral diseases of several crops transmitted by Polymyxa spp. (e.g., Polymyxa betae and Polymyxa graminis);
Diseases caused by bacteria:
   bacterial seedling blight of rice (Burkholderia plantarii), bacterial palea browning of rice (Pantoea ananatis), Bacterial leaf blight of rice (Xanthomonas oryzae pv. oryzae.), bacterial spot of cucumber (Pseudomonas syringae pv. lachrymans), bacterial wilt of eggplant (Ralstonia solanacearum), canker of citrus (Xanthomonas citri), bacterial soft rot of Chinese cabbage (Erwinia carotovora), scab of potato (Streptomyces scabiei), black leg of potato (Pectobacterium carotovorum and Dickeya sp.), bacterial leaf spot of peach (Pseudomona syringae, Erwinia nigrifluens, and Xanthomonas campestris), bacterial canker of Japanese apricot (Prunus mume) (Pseudomonas syringae pv. morsprunorum and Erwinia sp.), Goss's wilt of corn (Clavibacter michiganensis), Pierce's disease of grapes, olive, peach, and the like (Xylella fastidiosa), and crown gall of Rosaceae plants such as apple, peach, and cherries (Agrobacterium tumefaciens);
and the others.

The Present method for controlling a plant disease is carried out by applying an effective amount of the Present compound or the Composition A to a plant or soil for cultivating a plant. Examples of the treatment method include foliage treatment, soil treatment, and seed treatment.

The Present compound or the Composition A is usually used by mixing it with inert carrier(s) such as solid carrier(s), liquid carrier(s), and gaseous carrier(s), surfactant(s), and the like, and as needed, adding thereto auxiliary agent(s) for formulation such as binder(s), dispersant(s), and stabilizer(s) to be formulated into an aqueous suspension formulation, an oily suspension formulation, an oil solution, an emulsifiable concentrate, an emulsion formulation, a microemulsion formulation, a microcapsule formulation, a wettable powder, a granular wettable powder, a dust formulation, a granule, a tablet, an aerosol formulation, a resin formulation, or the like. In addition to these formulations, the Present compound or the Composition A may be used by formulating it into a dosage form described in Manual on development and use of FAO and WHO Specifications for pesticides, FAO Plant Production and Protection Papers- 271-276, prepared by the FAO/WHO Joint Meeting on Pesticide Specifications, 2016, ISSN:0259-2517.

These formulations usually comprise 0.0001% to 99% by weight ratio of the Present compound or the Composition A.

Examples of the solid carrier include fine powders and granules of clays (for example, pyrophyllite clay and kaolin clay), talc, calcium carbonate, diatomaceous earth, zeolite, bentonite, acid white clay, attapulgite, white carbon, ammonium sulfate, vermiculite, perlite, pumice, silica sand, chemical fertilizers (for example, ammonium sulfate, ammonium phosphate, ammonium nitrate, urea, and ammonium chloride), and the others; as well as resins (for example, polyethylene, polypropylene, polyester, polyurethane, polyamide, and polyvinyl chloride).

Examples of the liquid carrier include water, alcohols (for example, ethanol, cyclohexanol, benzyl alcohol, propylene glycol, and polyethylene glycol), ketones (for example, acetone and cyclohexanone), aromatic hydrocarbons (for example, xylene, phenyl xylyl ethane, and methylnaphthalene), aliphatic hydrocarbons (for example, hexane and cyclohexane), esters (for example, ethyl acetate, methyl oleate, and propylene carbonate), nitriles (for example, acetonitrile), ethers (for example, ethylene glycol dimethyl ether), amides (for example, N,N-dimethylformamide and N,N-dimethyloctanamide), sulfoxides (for example, dimethyl sulfoxide (hereinafter referred to as "DMSO")), lactams (for example, N-methylpyrrolidone and N-octylpyrrolidone), fatty acids (for example, oleic acid), and vegetable oils (for example, soybean oil).

Examples of the gaseous carrier include fluorocarbon, butane gas, LPG (liquefied petroleum gas), dimethyl ether, nitrogen, and carbon dioxide.

Examples of the surfactant include nonionic surfactants (for example, polyoxyethylene alkyl ethers, polyoxyethylene alkyl aryl ethers, and polyethylene glycol fatty acid esters), and anionic surfactants (for example, alkyl sulfonates, alkyl aryl sulfonates, and alkyl sulfates).

Examples of the other auxiliary agent for formulation include binders, dispersants, colorants, and stabilizers, and the specific examples thereof include polysaccharides (for example, starch, gum arabic, cellulose derivatives, and alginic acid), lignin derivatives, water-soluble synthetic polymers (for example, polyvinyl alcohol, polyvinyl pyrrolidone, and polyacrylic acids), acidic isopropyl phosphate, and dibutylhydroxytoluene.

Also, adjuvant(s) may be used as ingredient(s) for enhancing or assisting the efficacy of the Present compound. Specific examples thereof include Nimbus (registered trademark), Assist (registered trademark), Aureo (registered trademark), Iharol (registered trademark), Silwet L-77 (registered trademark), BreakThru (registered trademark), SundanceII (registered trademark), Induce (registered trademark), Penetrator (registered trademark), AgriDex (registered trademark), Lutensol A8 (registered trademark), NP-7 (registered trademark), Triton (registered trademark), Nufilm (registered trademark), Emulgator NP7 (registered trademark), Emulad (registered trademark), TRITON X 45 (registered trademark), AGRAL 90 (registered trademark), AGROTIN (registered trademark), ARPON (registered trademark), EnSpray N (registered trademark), and BANOLE (registered trademark).

In the present invention, examples of the plant include whole plant, stem and leaf, flower, ear, fruit, tree stem, branch, crown, seed, vegetative reproductive organ, and seedling.

A vegetative reproductive organ means a part of plant such as root, stem, and leaf which has a growth capability even when said part is separated from the plant body and placed into soil. Examples of the vegetative reproductive organ include tuberous root, creeping root, bulb, corm or solid bulb, tuber, rhizome, stolon, rhizophore, cane cuttings, propagule, and vine cutting. Stolon is also referred to as "runner", and propagule is also referred to as "propagulum" and categorized into broad bud and bulbil. Vine cutting means a shoot (collective term of leaf and stem) of sweet potato, glutinous yam, or the like. Bulb, corm or solid bulb, tuber, rhizome, cane cuttings, rhizophore, and tuberous root are also collectively referred to as "bulb". For example, cultivation of potato starts with planting a tuber into soil, and the tuber to be used is generally referred to as "seed potato".

Examples of a method for controlling plant diseases by applying an effective amount of the Present compound or the Composition A to soil include a method of applying an effective amount of the Present compound or the Composition A to soil before planting plants or after planting plants. Specifically, examples of the application method include planting hole treatment (for example, spraying into planting holes and soil mixing after planting hole treatment), plant foot treatment (for example, plant foot spraying, soil mixing after plant foot treatment, irrigation at plant foot, and plant foot treatment at a later seeding raising stage), planting furrow treatment (for example, planting furrow spraying and soil mixing after planting furrow treatment), planting row treatment (for example, planting row spraying, soil mixing after planting row treatment, and planting row spraying at a growing stage), planting row treatment at the time of sowing (for example, planting row spraying at the time of sowing and soil mixing after planting row treatment at the time of sowing), broadcast treatment (for example, overall soil surface spraying and soil mixing after broadcast treatment), side-article treatment, treatment of water surface (for example, application to water surface and application to water surface after flooding), other soil spraying treatment (for example, spraying of a granular formulation on leaves at a growing stage, spraying under a canopy or around a tree stem, spraying on the soil surface, mixing with surface soil, spraying into seed holes, spraying on the ground surfaces of furrows, and spraying between plants), other irrigation treatment (for example, soil irrigation, irrigation at a seedling raising stage, chemical solution injection treatment, irrigation of a plant part just above the ground, chemical solution drip irrigation, and chemigation), seedling raising box treatment (for example, spraying into a seedling raising box, irrigation of a seedling raising box, and flooding into a seedling raising box with chemical solution), seedling raising tray treatment (for example, spraying on a seedling raising tray, irrigation of a seedling raising tray, and flooding into a seedling raising tray with chemical solution), seedbed treatment (for example, spraying on a seedbed, irrigation of a seedbed, spraying on a lowland rice nursery, and immersion of seedlings), seedbed soil incorporation treatment (for example, mixing with seedbed soil, mixing with seedbed soil before sowing, spraying at sowing before covering with soil, spraying at sowing after covering with soil, and mixing with covering with soil), and other treatment (for example, mixing with culture soil, plowing under, mixing with surface soil, mixing with soil at the place where raindrops fall from a canopy, treatment at a planting position, spraying of a granule formulation on flower clusters, and mixing with a paste fertilizer).

Examples of the application to seeds (or seed treatments) include an application of the Present compound or the Composition A to seeds or vegetative reproductive organs, and specific examples thereof include spraying treatment in which a suspension of the Present compound or the Composition A is sprayed onto seed surface or the vegetative reproductive organ surface in the form of mist; smearing treatment in which the Present compound or the Composition A is coated on a surface of seeds or the vegetative reproductive organs; a soaking treatment in which the seeds or vegetative reproductive organs are soaked into the solution of the Present compound or the Composition A for a certain time; and a method for coating the seeds or the vegetative reproductive organs with a carrier containing the Present compound or the Composition A (for example, film coating treatment and pellet coating treatment). Examples of the above-described vegetative reproductive organ include particularly seed potato.

When the Composition A is applied to seeds or vegetative reproductive organs, the Composition A may be also applied to seeds or vegetative reproductive organs as a single formulation, or the Composition A may be applied to seeds or vegetative reproductive organs as multiple different formulations by multiple times. Examples of the method in which the Composition A is applied as multiple different formulations by multiple times include, for example, a method in which the formulations comprising as an active component the Present compound only are applied, and seeds or vegetative reproductive organs are air dried, followed by applying the formulations comprising the Present ingredient: and a method in which the formulations comprising as an active component the Present compound and the Present ingredients are applied, and seeds or vegetative reproductive organs are air dried, followed by applying the formulations comprising the Present ingredients other than the already-applied Present ingredients, are included.

As used herein, seeds or vegetative reproductive organs holding the Present compound or the Composition A mean seeds or vegetative reproductive organs in the state where the Present compound or the Composition A is adhered to a surface of the seeds or the vegetative reproductive organs. The above-described seeds or vegetative reproductive organs holding the Present compound or the Composition A may be adhered by any other materials that are different from the Present compound or the Composition A before or after being adhered the Present compound or the Composition A to the seeds or vegetative reproductive organs.

Also, when the Composition A is adhered in a form of layer(s) to a surface of seeds or vegetative reproductive organs, the layer(s) is/are composed of one layer or multiple layers. Also, when multiple layers are formed, each of the layer may be composed of a layer comprising one or more active ingredients, or a combination of a layer comprising one or more active ingredients and a layer not comprising an active ingredient.

Seeds or vegetative reproductive organs holding the Present compound or the Composition A can be obtained, for example, by applying the formulations comprising the Present compound or the Composition A by the above-described application method to seeds or vegetative reproductive organs.

The amount of the Present compound or the Composition A to be applied varies depending on the climate condition, the dosage form, the application period, the application method, the application site, diseases to be controlled, crops to be protected, and the like, and is usually within the range of 1 g to 500 g per 1000 m² of area for cultivating plants in case of foliage treatment or soil treatment. In case of seed treatment, the amount of the Present compound or the Composition A is usually within the range of 0.001 g to 100 g per 1 kg of seed. When the Present compound or the Composition A is formulated into an emulsifiable concentrate, a wettable powder, a flowable, or the like, such formulation is usually applied after diluting it with water in such a way that a concentration of the active ingredient is within the range of 0.01 to 10000 ppm, and then sparging it. In the case of being formulated into a dust formulation, a granule, or the like, such formulation is usually used as itself without diluting it.

The Present compound or the Composition A may be used as an agent for controlling plant diseases in croplands such as fields, paddy fields, grasses, and orchards. Examples of the plants include the followings.

corn (dent corn, flint corn, flour corn, popcorn, waxy corn, sweet corn, and field corn), rice (long grain rice, short grain rice, medium grain rice, japonica rice, tropical japonica rice, indica rice, javanica rice, paddy rice, upland rice, floating rice, direct-seeded rice, transplanted rice, and glutinous rice), wheat (bread wheat (hard wheat, soft wheat, medium wheat, red wheat, and white wheat), durum wheat, spelt wheat, and club wheat, winter wheat and spring wheat of them), barley (two-rowed barley (= barley for brewery), six-rowed barley, hull-less barley, and pearl barley, winter barley and spring barley of them), rye (winter rye and spring rye), triticale (winter triticale and spring triticale), oat (winter oat and spring oat), sorghum, cotton (upland cotton and Pima cotton), soybean (ripe seed harvest soybean, green soybeans, and early harvest soybeans, indeterminate type, determinate type, and semi-determinate type of them), peanut, buckwheat, beet (beets for sugar production, beets for feed, beets for root vegetable, beets for leaf vegetable, and beets for fuel), rapeseed (winter rapeseed and spring rapeseed), canola (winter canola and spring canola), sunflower (sunflowers for oil extraction, edible sunflowers, and sunflowers for ornamental purpose), sugar cane, tobacco, tea, mulberry, solanaceous vegetables (for example, eggplant, tomato, pimento, pepper, and potato), cucurbitaceous vegetables (for example, cucumber, pumpkin, zucchini, water melon, and melon), cruciferous vegetables (for example, Japanese radish, white turnip, horseradish, kohlrabi, Chinese cabbage, cabbage, leaf mustard, broccoli, and cauliflower), asteraceous vegetables (for example, burdock, crown daisy, artichoke, and lettuce), liliaceous vegetables (for example, welsh onion, onion, garlic, and asparagus), ammiaceous vegetables (for example, carrot, parsley, celery, and parsnip), chenopodiaceous vegetables (for example, spinach and Swiss chard), lamiaceous vegetables (for example, perilla, mint, and basil), strawberry, sweet potato, glutinous yam, eddoe, pomaceous fruits (for example, apple, pear, Japanese pear, Chinese white pear, Chinese quince, and quince), stone fleshy fruits (for example, peach, plum, nectarine, Japanese apricot (Prunus mume), cherry fruit, apricot, and prune), citrus fruits (for example, Citrus unshiu, orange, lemon, lime, and grapefruit), nuts (for example, chestnuts, walnuts, hazelnuts, almond, pistachio, cashew nuts, and macadamia nuts), berry fruits (for example, blueberry, cranberry, blackberry, and raspberry), grapes, Japanese persimmon, fig, olive, Japanese plum, banana, coffee, date palm, coconuts, ornamental plants, forest plants, turfs, grasses, and the others.

The above plants are not specifically limited as long as they are generally cultivated cultivars. The above plants also include plants which may be produced by natural breeding, plants which may be generated by mutation, F1 hybrid plants, and genetically modified crops. Examples of the genetically modified crops include plants which have resistance to HPPD (4-hydroxyphenylpyruvate dioxygenase enzyme) inhibitors such as isoxaflutole, ALS (acetolactate synthase) inhibitors such as imazethapyr and thifensulfuron-methyl, EPSP (5-enolpyruvylshikimate-3-phosphate synthase) inhibitors, glutamine synthetase inhibitors, PPO (protoporphyrinogen oxidase) inhibitors, or herbicide such as bromoxynil and dicamba; plants which can synthesize a selective toxin known in Bacillus spp. such as Bacillus thuringiensis or the like; and plants which can synthesize a gene fragment or the like which is partially identical to an endogenous gene derived from a harmful insect, and induce a gene silencing (RNAi; RNA interference) in the target harmful insect to achieve a specific insecticidal activity.

### EXAMPLES

Hereinafter, the present invention is illustrated more in detail by a Preparation Example, Formulation Examples, Test Examples, and the like, but the present invention is not limited to these Examples only.

First, a Preparation Example of the Present compound is shown below.

In the following Example, quantitative analysis was carried out by using high performance liquid chromatography, unless otherwise described. The analysis conditions are as follows.

### [Analysis conditions of high performance liquid chromatography]

Mobile phase: Solution A: 5 mM aqueous solution of ammonium carbonate, Solution B: acetonitrile
Column: XBridge (trademark) Phenyl, particle size: 3.5 µm, 4.6 mm I.D. × 150 mm (manufactured by Waters)
UV measurement wavelength: 250 nm
Flow rate: 1.0 mL/min
Column oven: 30°C
Pump: two LC-20AD (manufactured by Shimadzu Corporation)
(high-pressure gradient)
Gradient conditions: sending a solution with the concentration gradient described in Table LC1.

**Table LC1**

| Time (min) | Solution A (%) | Solution B (%) |
|---|---|---|
| 0 | 70 | 30 |
| 15 | 60 | 40 |
| 30 | 60 | 40 |
| 60 | 20 | 80 |
| 65 | 20 | 80 |

A compound represented by formula (3) 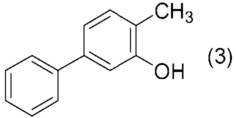 is commercially available, or may also be prepared by using known method(s).

### Reference Preparation Example 1

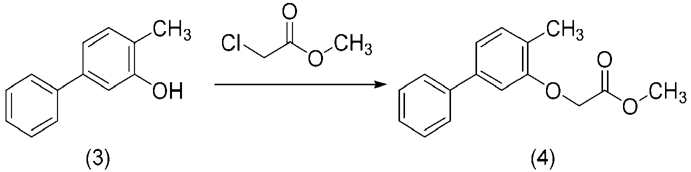

To a mixture of the compound represented by formula (3) (45.8 g), xylene (137 g), and methyl chloroacetate (43.2 g) was added dropwise a 28% by weight solution of sodium methoxide in methanol (72.0 g) at 80°C, and the resulting mixture was stirred at the same temperature for 1 hour. The resulting mixture was cooled to 60°C, dimethyl sulfate (3.4 g) was added thereto, and the resulting mixture was stirred at the same temperature for 3 hours. To the resulting mixture was added water at 60°C to separate the mixture, and the resulting organic layer was concentrated under reduced pressure. To the concentrated residue were sequentially added methanol and water to precipitate crystals, and the compound represented by formula (4) was isolated as crystals (59.8 g).

### Preparation Example 1

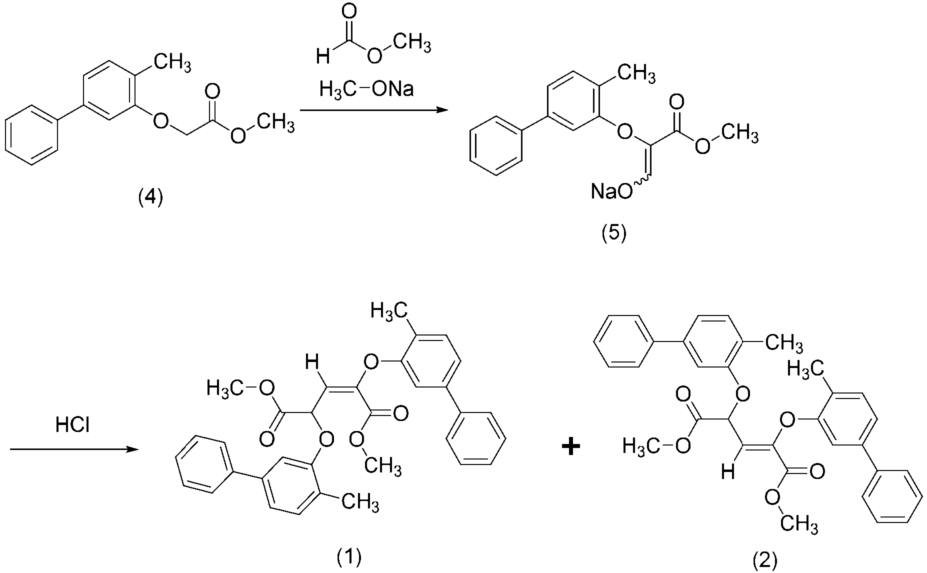

To a mixture of xylene (10.0 g) and sodium methoxide (4.2 g) was added dropwise a mixture of the compound represented by formula (4) (10.0 g) prepared according to the method described in the Reference Preparation Example 1, methyl formate (4.7 g), tetrahydrofuran (20.0 g), and xylene (10.0 g) at 30°C over 3 hours. The resulting mixture was cooled to 10°C, and water (30.0 g) and 35% by weight hydrochloric acid (1.33 g) were sequentially added thereto. The resulting mixture was separated to give an aqueous layer (60.0 g) comprising the compound represented by formula (5).

To the resulting above aqueous layer (60.0 g) were sequentially added xylene (30.0 g) and tetrabutylammonium chloride (1.08 g) at room temperature. The resulting mixture was stirred at room temperature and 35% by weight hydrochloric acid (2.0 g) was added dropwise thereto with stirring. The resulting mixture was stirred at 80°C for 20 hours, then cooled to room temperature, separated, and the resulting organic layer was concentrated under reduced pressure. The resulting concentrated residue was analyzed by high performance liquid chromatography to confirm that the retention time of the compound represented by formula (1) was 58.2 minutes and the retention time of the compound represented by formula (2) was 57.5 minutes under the above analysis conditions of high performance liquid chromatography. The above concentrated residue was purified by silica gel column chromatography (hexane/ethyl acetate solvent system) to give the compound represented by formula (2) (1.89 g) as solids.

The values of ¹H-NMR of the compound represented by formula (2) are shown below.
¹H-NMR (CDCl₃) δ: 7.47-7.44 (2H, m), 7.44-7.41 (2H, m), 7.39-7.35 (4H, m), 7.32-7.28 (2H, m), 7.23 (1H, d), 7.18 (2H, dd), 7.10 (1H, dd), 7.04 (1H, d), 6.90 (1H, d), 6.68 (1H, d), 5.78 (1H, d), 3.71 (6H, s), 2.36 (3H, s), 2.32 (3H, s).

Next, Formulation Examples of the Present compound are shown below. The "part(s)" represents "part(s) by weight".

### Formulation Example 1

The Present compound (20 parts), a mixture of white carbon and polyoxyethylene alkyl ether sulfate ammonium salt (weight ratio of 1 : 1) (35 parts), and water (45 parts) are mixed, and the resulting mixture is treated with a grinding mill to obtain a formulation.

### Formulation Example 2

A mixture of polyoxyethylene alkyl ether sulfate ammonium salt and silica (weight ratio of 1 : 1) (35 parts), the Present compound (10 parts), and water (55 parts) are mixed, and the resulting mixture is subjected to fine grinding according to a wet grinding method to obtain a formulation.

### Formulation Example 3

The Present compound (50 parts), calcium lignin sulfonate (3 parts), sodium lauryl sulfate (2 parts), and silica (45 parts) are ground and mixed to obtain a formulation.

### Formulation Example 4

The Present compound (5 parts), polyoxyethylene styryl phenyl ether (9 parts), polyoxyethylene decyl ether (number of added ethylene-oxide: 5) (5 parts), calcium dodecylbenzene sulfonate (6 parts), and xylene (75 parts) are mixed to obtain a formulation.

### Formulation Example 5

The Present compound (2 parts), silica (1 part), calcium lignin sulfonate (2 parts), bentonite (30 parts), and kaolin clay (65 parts) are ground and mixed, an appropriate amount of water is added thereto, the resulting mixture is kneaded, subjected to granulation with a granulator, and then dried to obtain a formulation.

### Formulation Example 6

The Present compound (10 parts), and a mixture of benzyl alcohol (18 parts) and DMSO (9 parts) are mixed, GERONOL (registered trademark) TE250 (6.3 parts), Ethylan (registered trademark) NS-500LQ (2.7 parts), and solvent naphtha (54 parts) are added thereto, and the resulting mixture is mixed to obtain a formulation.

Further, the control efficacy of the Present compound against plant diseases is shown by Test Examples.

The term of "untreated" in Test Examples 1 and 2 means that foliage application of a diluted solution with water of a formulation comprising the Present compound was not carried out. Also, the term of "formulated Present compound" in Test Examples 1 and 2 represents a formulation obtained by mixing the Present compound (20 parts), a mixture of white carbon and polyoxyethylene alkyl ether sulfate ammonium salt (weight ratio of 1 : 1) (35 parts), and water to obtain a mixture (total amount: 100 parts), and treating the resulting mixture with a grinding mill. Further, the term of "Present compound" in Test Examples 1 and 2 represents the compound represented by formula (2) prepared according to the production method described in the Preparation Example 1.

### Test Example 1 Control test against barley net blotch

### (Pyrenophora teres)

A plastic pot was filled with soil, barley (cultivar: Nishinohoshi) was seeded thereto, and grown in a greenhouse for 7 days. A formulated Present compound was mixed with water in such a way that the concentration thereof was 200 ppm, and the resulting mixture was sprayed to foliage of the above barley so as to adequately adhere onto the surfaces of leaves of the above barley. After spraying the mixture, the barley was air-dried. After 1 day, an aqueous suspension of spores of Pyrenophora teres was inoculated by spraying to the barley. After the inoculation, the barley was placed in a greenhouse at 23°C in daytime and at 20°C in nighttime under a humid condition for 3 days, then cultured in a greenhouse for 7 days, and then a lesion area was investigated. As a result, the lesion area in the barley treated with the Present compound was 30% or less relative to the lesion area in the untreated barley.

### Test Example 2 Control test against soybean rust

### (Phakopsora pachyrhizi)

A plastic pot was filled with soil, soybeans (cultivar: Kurosengoku) were seeded thereto, and cultured and grown in a greenhouse for 10 to 14 days. A formulated Present compound was mixed with water in such a way that the concentration thereof was 800 ppm, and the resulting mixture was sprayed to foliage of the above soybeans so as to adequately adhere onto the surfaces of leaves of the above soybeans. After spraying the mixture, the soybeans were air-dried. After 2 to 5 days, an aqueous suspension of spores of Phakopsora pachyrhizi was inoculated by spraying to the soybeans. After the inoculation, the soybeans were placed in a greenhouse at 23°C in daytime and at 20°C in nighttime under a humid condition for 1 to 2 day(s), then cultured in a greenhouse for 10 to 14 days, and then a lesion area was investigated. As a result, the lesion area in the soybeans treated with the Present compound was 30% or less relative to the lesion area in the untreated soybeans.

### INDUSTRIAL APPLICABILITY

The Present compound has excellent control effects against plant diseases, and is useful as an active ingredient of a composition for controlling plant diseases.

## Claims

1. A compound represented by formula (A)

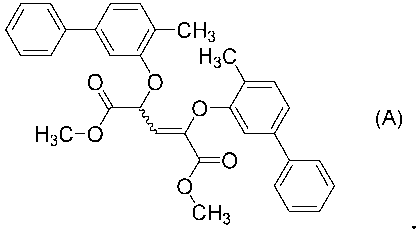

2. A composition for controlling a plant disease comprising the compound according to claim 1 and an inert carrier.

3. A composition comprising one or more ingredient(s) selected from the group consisting of Group (a), Group (b), Group (c), and Group (d), and the compound according to claim 1:
Group (a): a group consisting of insecticidal active ingredients, miticidal active ingredients, and nematicidal active ingredients;
Group (b): fungicidal active ingredients;
Group (c): plant growth regulatory ingredients;
Group (d): repellent ingredients.

4. A method for controlling a plant disease which comprises applying an effective amount of the compound according to claim 1, an effective amount of the composition for controlling a plant disease according to claim 2, or an effective amount of the composition according to claim 3, to a plant or soil for cultivating a plant.

5. Use of the compound according to claim 1, the composition for controlling a plant disease according to claim 2, or the composition according to claim 3, for controlling a plant disease.

6. A seed or a vegetative reproductive organ holding an effective amount of the compound according to claim 1, an effective amount of the composition for controlling a plant disease according to claim 2, or an effective amount of the composition according to claim 3.
